# EUROPEAN PATENT APPLICATION

(11) **EP 4 176 935 A1**
(43) Date of publication of application: **10.05.2023**
(21) Application number: 20942463.9
(22) Date of filing: 29.06.2020
(51) Int. Cl.: A61P 19/02, A61K 36/736

(54) **QUALITY-OF-LIFE IMPROVING AGENT**

(71) Applicant: Nihon Sizen Hakkoh Co., Ltd., Takayama-shi, Gifu 501-5401 (JP)
(72) Inventor: HIGASHI, Naoki, Tokyo 145-0071 (JP); NAKANISHI, Masahiro, Takayama-shi, Gifu 501-5401 (JP); ADACHI, Teruki, Takayama-shi, Gifu 501-5401 (JP)
(74) Representative: Blodig, Wolfgang
(86) International application number: PCT/JP2020/025433
(87) International publication number: WO 2022/003749

(57) **Abstract**

A quality-of-life improving agent, characterized by containing, as an active ingredient, a plant fermented product that is a mixture of the following substances (a) to (g): (a) a koji mold fermented product of one type or two or more types of beans/grains selected from the group consisting of barley, black soybean, red rice, black rice, red bean, adlay, Japanese barnyard millet, foxtail millet, and proso millet; (b) a yeast and/or lactic acid bacterium fermented product of one type or two or more types of fruits selected from the group consisting of mandarin orange, grape, apple, crimson glory vine, peach, persimmon, papaya, pear, watermelon, Prunus mume, fig, Chinese quince, pumpkin, kumquat, yuzu, loquat, apricot, jujube, chestnut, silver vine, and Prunus salicina; (c) a yeast and/or lactic acid bacterium fermented product of one type or two or more types of root vegetables/tubers selected from the group consisting of purple sweet potato, Jerusalem artichoke, carrot, onion, sweet potato, taro, wild yam, daikon, red turnip, burdock, lotus root, yacon, lily bulb, threeleaf arrowhead, ginger, garlic, and turmeric; (d) a yeast and/or lactic acid bacterium fermented product of one type or two or more types of flowers/leafy vegetables selected from the group consisting of cabbage, shiso, mulberry leaf, fish mint, mugwort, Sasa veitchii, and dandelion; (e) a yeast and/or lactic acid bacterium fermented product of one type or two or more types of seaweeds selected from the group consisting of kombu, wakame, and mozuku; (f) a yeast and/or lactic acid bacterium fermented product of one type or two or more types of seeds selected from the group consisting of black sesame, walnut, and ginkgo nut; and (g) a yeast and/or lactic acid bacterium fermented product of one type or two types of mushrooms selected from the group consisting of hen-of-the-woods and shiitake, can improve a wide range of quality of life related to emotions/behaviors, geriatric symptoms, digestive symptoms, chronic fatigue, chronic inflammation, metabolic diseases, and the like.

## Description

### Technical Field

The present invention relates to a quality-of-life improving agent using a plant fermented product.

### Background Art

It has been a long time since QOL (quality of life) came to be emphasized in the medical field, and for example, SHIMOZUMA has explained the history of QOL (NPL 1). According to this, the idea of QOL starts from the treatment of cancer, and aims to eliminate various side effects or discomforts caused by a treatment of various cancers aiming at medical healing.

As described above, the target range of QOL is wide, but further, the avoidance and elimination of discomforts are also targeted, and various and complex symptoms ranging from predisease to disease are targeted.

In addition to the above QOL, with the rapid progress of aging, there is an increasing need for improvement of the decrease in QOL due to the aging of body organs, tissues, or cells themselves and regulatory functions.

However, a medicinal product based on science developed in the West is required to be able to explain the cause of disease and symptoms in a 1:1 relationship, and Western science is the main starting point also for Japan's medical system. The basis of Western medicine is that the diagnosis of the disease is confirmed and the prescription therefor is determined. Therefore, such a medicinal product could not have been able to comprehensively improve a wide range of QOL.

### Citation List

### Patent Literature

PTL 1: Japanese Patent No. 6013670
PTL 2: WO 2019/009437
PTL 3: WO 2019/009438

### Non Patent Literature

NPL 1: "History and Future Perspective of QOL Assessment Research" Kojiro SHIMOZUMA, Japanese Journal of Behavioral Medicine, Vol. 21, (1) 4-7 (2015)

### Summary of Invention

### Technical Problem

Therefore, an object of the present invention is to provide a quality-of-life improving agent capable of comprehensively improving a wide range of QOL.

### Solution to Problem

As a result of intensive studies to achieve the above object, the present inventors found that a plant fermented product, which has been developed so far by the present inventors, and whose anti-aging action (PTL 1), spontaneous cancer preventive action (PTL 2), and immune checkpoint suppress action (PTL 3) have been revealed, can improve a wide range of QOL, and thus completed the present invention.

That is, the present invention is directed to a quality-of-life improving agent, characterized by containing, as an active ingredient, a plant fermented product that is a mixture of the following substances (a) to (g):
(a) a koji mold fermented product of one type or two or more types of beans/grains selected from the group consisting of barley, black soybean, red rice, black rice, red bean, adlay, Japanese barnyard millet, foxtail millet, and proso millet;
(b) a yeast and/or lactic acid bacterium fermented product of one type or two or more types of fruits selected from the group consisting of mandarin orange, grape, apple, crimson glory vine, peach, persimmon, papaya, pear, watermelon, Prunus mume, fig, Chinese quince, pumpkin, kumquat, yuzu, loquat, apricot, jujube, chestnut, silver vine, and Prunus salicina;
(c) a yeast and/or lactic acid bacterium fermented product of one type or two or more types of root vegetables/tubers selected from the group consisting of purple sweet potato, Jerusalem artichoke, carrot, onion, sweet potato, taro, wild yam, daikon, red turnip, burdock, lotus root, yacon, lily bulb, threeleaf arrowhead, ginger, garlic, and turmeric;
(d) a yeast and/or lactic acid bacterium fermented product of one type or two or more types of flowers/leafy vegetables selected from the group consisting of cabbage, shiso, mulberry leaf, fish mint, mugwort, Sasa veitchii, and dandelion;
(e) a yeast and/or lactic acid bacterium fermented product of one type or two or more types of seaweeds selected from the group consisting of kombu, wakame, and mozuku;
(f) a yeast and/or lactic acid bacterium fermented product of one type or two or more types of seeds selected from the group consisting of black sesame, walnut, and ginkgo nut; and
(g) a yeast and/or lactic acid bacterium fermented product of one type or two types of mushrooms selected from the group consisting of hen-of-the-woods and shiitake.

Further, the present invention is directed to a food or drink for improving the quality of life, containing the quality-of-life improving agent.

### Advantageous Effects of Invention

The quality-of-life improving agent of the present invention contains a specific plant fermented product having a rich history of being eaten as an active ingredient, and therefore is suitable also for being prepared as a food or drink, or the like.

In addition, the quality-of-life improving agent of the present invention is different from the conventional ones, and can comprehensively improve a wide range of QOL, for example, QOL based on emotions/behaviors, geriatric symptoms, digestive symptoms, chronic fatigue, chronic inflammation, metabolic diseases, dementia, or the like.

### Brief Description of Drawings

[FIG. 1] FIG. 1 shows heat maps of DNA microarray analysis of the thymus and the kidney.
[FIG. 2] FIG. 2 shows the abundance ratio at the genus level of enteric bacteria in senescence-accelerated mice.
[FIG. 3] FIG. 3 shows an increase or decrease in the genus Lactobacillus in senescence-accelerated mice.
[FIG. 4] FIG. 4 shows the number of the genus Lactobacillus in senescence-accelerated mice in each test group.
[FIG. 5] FIG. 5 shows an increase or decrease in the genus Bacteroides in senescence-accelerated mice.
[FIG. 6] FIG. 6 shows an increase or decrease in the genus Clostridium in senescence-accelerated mice.
[FIG. 7] FIG. 7 shows the results of α-diversity using Unifrac analysis.
[FIG. 8] FIG. 8 shows the results of β-diversity using Unifrac analysis.
[FIG. 9] FIG. 9 shows the results of Weighted Unifrac analysis in which the number of reads (composition ratio) between bacterial floras was taken into consideration.
[FIG. 10] FIG. 10 shows the T lymphocyte age of a test subject immediately before ingestion.
[FIG. 11] FIG. 11 shows the T lymphocyte age of the test subject after 4-weeks ingestion.
[FIG. 12] FIG. 12 shows the T lymphocyte age of the test subject 2 weeks after the completion of ingestion.

### Description of Embodiments

The quality-of-life improving agent of the present invention contains the following plant fermented product as an active ingredient. This plant fermented product is a mixture of the following plant fermented products (a) to (g).
(a) a fermented product obtained by allowing a koji mold to act on one type or two or more types of beans/grains selected from the group consisting of barley, black soybean, red rice, black rice, red bean, adlay, Japanese barnyard millet, foxtail millet, and proso millet [0017]
(b) a fermented product obtained by allowing a yeast and/or a lactic acid bacterium to act on one type or two or more types of fruits selected from the group consisting of mandarin orange, grape, apple, crimson glory vine, peach, persimmon, papaya, pear, watermelon, Prunus mume, fig, Chinese quince, pumpkin, kumquat, yuzu, loquat, apricot, jujube, chestnut, silver vine, and Prunus salicina
(c) a fermented product obtained by allowing a yeast and/or a lactic acid bacterium to act on one type or two or more types of root vegetables/tubers selected from the group consisting of purple sweet potato, Jerusalem artichoke, carrot, onion, sweet potato, taro, wild yam, daikon, red turnip, burdock, lotus root, yacon, lily bulb, threeleaf arrowhead, ginger, garlic, and turmeric
(d) a fermented product obtained by allowing a yeast and/or a lactic acid bacterium to act on one type or two or more types of flowers/leafy vegetables selected from the group consisting of cabbage, shiso, mulberry leaf, fish mint, mugwort, Sasa veitchii, and dandelion
(e) a fermented product obtained by allowing a yeast and/or a lactic acid bacterium to act on one type or two or more types of seaweeds selected from the group consisting of kombu, wakame, and mozuku
(f) a fermented product obtained by allowing a yeast and/or a lactic acid bacterium to act on one type or two or more types of seeds selected from the group consisting of black sesame, walnut, and ginkgo nut
(g) a fermented product obtained by allowing a yeast and/or a lactic acid bacterium to act on one type or two types of mushrooms selected from the group consisting of hen-of-the-woods and shiitake

In the quality-of-life improving agent of the present invention, a plant fermented product obtained by mixing the fermented products (a) to (g) may be used as it is as an active ingredient, but by further performing multi-step fermentation, the taste and pharmaceutical properties can be improved.

Examples of the yeast include yeasts belonging to the genus Saccharomyces, the genus Zygosaccharomyces, and the like, and among them, Saccharomyces cerevisiae (S. cervisiae), Zygosaccharomyces rouxii (Z. rouxii), Saccharomyces exiguus (S. exiguus), or the like is preferably used. Examples of the lactic acid bacterium include lactic acid bacteria belonging to the genus Pediococcus, the genus Leuconostoc, the genus Lactobacillus, the genus Lactococcus, and the like, and among them, Pediococcus acidilacti (P. acidilacti), Lactobacillus brevis (L. brevis), Leuconostoc mesenteroides (L. mesenteroides), Lactobacillus plantarum (L. plantarum), Lactobacillus lactis (L. lactis), Lactobacillus sake! (L. sakei), Pediococcus pentosaceus (P. pentosaceus), Lactobacillus casei (L. casei), Lactobacillus reuteri (L. reuteri), Lactobacillus curvatus (L. curvatus), or the like is preferably used, and one strain or two or more strains of these can be used. As the koji mold, Aspergillus oryzae, Aspergillus niger, Aspergillus kawachii, and the like can be exemplified, and one strain or two or more strains of these can be used. As these, commercially available ones can also be used.

A plant such as a bean/grain or a fruit to be fermented may be used as it is, or may be subjected to a pretreatment such as crushing or drying as needed. Further, it may be diluted by adding water thereto as needed.

The fermented products (a) to (g) described above are obtained by inoculating a lactic acid bacterium, a yeast, or a koji mold into a plant as a raw material, followed by culturing. The culturing may be performed by a conventional method. For example, a lactic acid bacterium, a yeast, or a koji mold is added in an amount of about 0.001 to 1 mass% to one type of plant or a mixture of two or more types of plants, and a fermentation treatment may be performed at 20 to 50°C for about 70 to 140 hours.

The thus obtained fermented products (a) to (g) are mixed and the resultant can be used as it is as an active ingredient, but it is preferred to perform additional culturing at 20 to 40°C for about 200 to 300 hours as needed. Further, it is preferred that the mixture is subjected to post-fermentation (aging). In the post-fermentation, an acetic acid bacterium may be allowed to act as needed, and for example, an acetic acid fermented product obtained by allowing an acetic acid bacterium such as Acetobacter aceti (A. aceti) to act may be added to a material obtained by allowing the above-mentioned yeast to act on one type or two or more types of plants included in the above (a) to (g). The post-fermentation may be performed at 25 to 35°C for 70 hours to about 1 year or so. By undergoing the aging process through the post-fermentation, the taste and pharmaceutical properties can be improved, and further, the antioxidative activity can also be enhanced.

As an example of a preferred method for producing the above-mentioned plant fermented product, a multi-stage complex fermentation method is exemplified. In this method, a complex lactic acid bacterium fermented product containing, as main raw materials, fruits, vegetables, and seaweeds, and a yeast fermented product containing, as main raw materials, vegetables, root vegetables, seeds, mushrooms, and fruits are mixed, and a koji mold fermented product containing, as main raw materials, grains and beans is added thereto, and further an acetic acid fermented product is added thereto, followed by mixing, and then filtration concentration is performed, and further post-fermentation is performed for about 1 year or so. It is considered that by such multi-stage complex fermentation, each fermented product is further assimilated and converted by another type of strain, thereby improving the flavor and also enhancing an effect such as an antioxidative activity.

The plant fermented product of the active ingredient used in the present invention has the following properties (1) to (4).

### (1) Taste

It has sweetness derived from raw materials such as fruits and vegetables, and sweetness derived from koji in addition to organic acids. It contains polyphenols derived from raw materials, but has little bitterness.

### (2) Solubility in water

It dissolves easily in water.

### (3) Stability

It is stable against heat and an acid, and the paste does not spoil or change in taste even when it is stored at room temperature for 1 year.

### (4) Safety

The vegetables, fruits, herbs, etc. used as raw materials are eaten daily, and as the yeast, the lactic acid bacterium, and the koji mold used for fermentation, strains derived from brewing of foods, or pickles, or the like are used, and therefore, they have a rich history of being eaten.

The plant fermented product used in the present invention has the following properties (A) to (D).

### (A) General ingredients (per 100 g)

| | |
|---|---|
| moisture | 15 to 35 g |
| protein | 5 to 20 g |
| lipid | 1 to 8 g |
| ash content | 1 to 5 g |
| carbohydrate | 30 to 70 g |
| sodium | 40 to 150 mg |
| vitamin B6 | 0.1 to 0.5 mg |
| energy | 200 to 500 kcal |

### (B) Amino acid composition (per 100 g)

| | |
|---|---|
| arginine | 0.2 to 0.6 g |
| lysine | 0.1 to 0.7 g |
| histidine | 0.1 to 0.4 g |
| phenylalanine | 0.2 to 0.8 g |
| tyrosine | 0.1 to 0.6 g |
| leucine | 0.3 to 1.2 g |
| isoleucine | 0.2 to 0.8 g |
| methionine | 0.05 to 0.3 g |
| valine | 0.2 to 0.9 g |
| alanine | 0.2 to 0.9 g |
| glycine | 0.2 to 0.7 g |
| proline | 0.4 to 1.2 g |
| glutamic acid | 1.2 to 3.0 g |
| serin | 0.2 to 0.8 g |
| threonine | 0.2 to 0.7 g |
| aspartic acid | 0.4 to 1.5 g |
| tryptophan | 0.03 to 0.15 g |
| cystine | 0.05 to 0.40 g |

### (C) Organic acid composition (per 100 g)

| | |
|---|---|
| citric acid | 0.5 to 1.2 g |
| malic acid | 0.05 to 0.5 g |
| succinic acid | 0.04 to 0.3 g |
| lactic acid | 0.5 to 6.0 g |
| formic acid | 0.01 to 0.1 g |
| pyruvic acid | 0.005 to 0.05 g |
| free γ-aminobutyric acid | 0.01 to 0.05 g |

### (D) Mineral composition (per 100 g)

| | |
|---|---|
| phosphorus | 100 to 400 mg |
| iron | 1 to 5 mg |
| calcium | 500 to 900 mg |
| potassium | 600 to 1000 mg |
| magnesium | 70 to 120 mg |
| zinc | 0.8 to 1.6 mg |
| iodine | 1.0 to 2.5 mg |

The quality-of-life improving agent containing this plant fermented product as an active ingredient can improve various QOL, for example, QOL based on emotions/behaviors, geriatric symptoms, digestive symptoms, chronic fatigue, chronic inflammation, metabolic diseases, dementia, or the like. In addition, by ingesting this quality-of-life improving agent, it is possible to reduce the dose or terminate a medication in use in various categories.

The quality-of-life improving agent of the present invention is obtained by adding a pharmacologically acceptable carrier, excipient, water activity adjusting agent, or the like to the thus obtained plant fermented product and formulating the resulting mixture according to a known pharmaceutical production method. The plant fermented product may be concentrated to adjust the concentration or may be powdered by spray- or freeze-drying or the like as needed. As the carrier or the excipient used in the formulation, for example, lactose, glucose, sucrose, starch, a sugar mixture, or the like is used. As the final form, a solution, a paste, a soft capsule, a chewable, or a capsule is used. As the dosage and administration, for example, a preparation may be orally ingested so that the daily dose per adult of the plant fermented product which is the active ingredient is about 0.1 g or more, preferably 0.1 to 12 g, more preferably about 0.6 to 10 g (in terms of solid content).

Further, the quality-of-life improving agent of the present invention can be used in a medicinal product, a quasi-drug, or the like, and can also be formed into a food or drink by blending together with a known food material. The above-mentioned plant fermented product can be ingested as it is, but in order to improve the shelf life, it may be filtered or concentrated after sterilization, or an excipient is added thereto as needed and the resultant may be formed into a powder by spray- or freeze-drying. Further, in order to improve the shelf life in the distribution process, one with a water activity reduced by concentration is desired. As the form of such a food or drink, a paste, a soft capsule, a tablet, a drink, and the like can be exemplified. By orally ingesting the plant fermented product at a daily dose per adult of about 0.1 g or more, preferably 0.1 to 12 g, more preferably about 0.6 to 10 g (in terms of solid content), an excellent QOL improving effect or the like can be obtained. Examples of a commercially available product obtained by formulating such a plant fermented product into a preparation or a food include Tensei Koso Kinjirushi, Tensei Koso Capsule, and Amo Koso (manufactured by NIHON SIZEN HAKKOH CO., LTD.).

Further, the food or drink containing the quality-of-life improving agent of the present invention may be, in addition to the above-mentioned food or drink, one obtained by using the plant fermented product which is the active ingredient as a kind of seasoning or the like and incorporating it in a seasoning such as miso, a bread such as a pan loaf, confectionery or a snack such as a rice cracker, a cookie, chocolate, a candy, a steamed bun, or a cake, a dairy product such as yogurt or cheese, a pickle such as takuwan, a noodle such as ramen, soba, or udon, a soup such as corn potage or a wakame soup, or a food or drink such as a soft drink, a health drink, a carbonated drink, or a fruit juice drink.

Further, the quality-of-life improving agent of the present invention can improve QOL based on dementia such as Alzheimer's disease, but in this case, for example, it is preferred to combine it with a known substance confirmed to have a dementia improving action. Examples of such a substance include anserine derived from fish. Specifically, when the quality-of-life improving agent of the present invention is combined with anserine, anserine may be orally ingested at a daily dose per adult of 500 to 1500 mg, preferably 700 to 1000 mg simultaneously with the daily dose of the quality-of-life improving agent of the present invention. Note that the quality-of-life improving agent of the present invention can improve QOL based on dementia such as Alzheimer's disease, but needless to say, it can also prevent dementia if it is ingested before developing dementia such as Alzheimer's disease.

Still further, the plant fermented product which is the active ingredient of the quality-of-life improving agent of the present invention can improve QOL based on chronic inflammation. It is shown in the present description that the plant fermented product is different from a medicinal product, and broadly suppresses the expression of an inflammatory cytokine or a chemokine, particularly the expression of IL-6. Therefore, in particular, the plant fermented product can also be used for prevention or treatment of various diseases associated with chronic inflammation caused by the overexpression or continuous expression of IL-6. Examples of such a disease include rheumatoid arthritis, juvenile idiopathic arthritis, Castleman's disease, adult-onset Still's disease, and cytokine storm, and in particular, it can suppress cytokine storm due to new coronavirus (COVID-19) infection (acute exacerbation of multi-organ failure, or the like), Kawasaki disease-like symptoms, sepsis, Behcet's disease, psoriasis, or the like. In order to use the plant fermented product for suppressing the expression of IL-6, it may be administered at the same daily dose as the quality-of-life improving agent of the present invention.

### Examples

Hereinafter, the present invention will be described in detail with reference to Examples, but the present invention is by no means limited to these Examples.

### Production Example 1

Production of plant fermented product:
The following plants were used as raw materials.
   (a) beans and grains (barley, black soybean, red rice, black rice, red bean, adlay, Japanese barnyard millet, foxtail millet, and proso millet)
   (b) fruits (mandarin orange, grape, apple, crimson glory vine, peach, persimmon, papaya, pear, watermelon, Prunus mume, fig, Chinese quince, pumpkin, kumquat, yuzu, loquat, apricot, jujube, chestnut, silver vine, and Prunus salicina)
   (c) root vegetables (purple sweet potato, Jerusalem artichoke, carrot, onion, sweet potato, taro, wild yam, daikon, red turnip, burdock, lotus root, yacon, lily bulb, threeleaf arrowhead, ginger, garlic, and turmeric)
   (d) flowers and leafy vegetables (cabbage, shiso, mulberry leaf, fish mint, mugwort, Sasa veitchii, and dandelion)
   (e) seaweeds (kombu, wakame, and mozuku)
   (f) seeds (black sesame, walnut, and ginkgo nut)
   (g) mushrooms (hen-of-the-woods and shiitake)

A culture solution prepared so that the cell density of lactic acid bacteria (P. acidilacti, L. brevis, L. mesenteroides, L. plantarum, L. lactis, L. sakei, and L. casei) was about 1.0 × 10⁵ cfu/g was inoculated at 0.2 mass% in the raw materials (730 g) of the above-mentioned (c), (d) and (g), and cultured at 30°C for 50 hours. Further, a culture solution prepared so that the cell density of yeasts (5 strains of S. cervisiae and 2 strains of Z. rouxii) was about 1.0 × 10⁵ cfu/g was inoculated at 0.4 mass% in the raw materials (900 kg) of the above-mentioned (b), (e), and (f), and cultured at 30°C for 50 hours. In the bean and grain-based raw material (a) (1000 kg), koji molds (yellow koji mold, black koji mold, and white koji mold) were inoculated at 0.1 mass%, and cultured at 35°C for 70 hours. Then, the respective cultures were mixed, and thereafter, the resultant was cultured at 30°C for 200 hours. The moromi obtained after completion of fermentation was subjected to a solid-liquid separation operation, and the obtained filtrate was concentrated to form a paste, and the paste was dispensed into a container, which was further fermented (aged) for one year, whereby a plant fermented product was obtained.

The plant fermented product obtained in Production Example 1 had the following properties.

### (A) General analysis value (per 100 g)

| | |
|---|---|
| moisture | 25.2 g |
| protein | 11.8 g |
| lipid | 3.6 g |
| ash content | 2.1 g |
| carbohydrate | 57.3 g |
| sodium | 54.0 mg |
| vitamin B6 | 0.20 mg |
| energy | 309 kcal |

### (B) Amino acid composition (per 100 g)

The sample was hydrolyzed with 6 N hydrochloric acid and the resultant was analyzed by an automatic amino acid analyzer. For cystine, hydrochloric acid hydrolysis was used after a performic acid oxidation treatment. For tryptophan, high-speed liquid chromatography was used.

| | |
|---|---|
| arginine | 0.33 g |
| lysine | 0.34 g |
| histidine | 0.22 g |
| phenylalanine | 0.51 g |
| tyrosine | 0.32 g |
| leucine | 0.74 g |
| isoleucine | 0.42 g |
| methionine | 0.13 g |
| valine | 0.54 g |
| alanine | 0.48 g |
| glycine | 0.42 g |
| proline | 0.92 g |
| glutamic acid | 2.25 g |
| serin | 0.4 g |
| threonine | 0.36 g |
| aspartic acid | 0.84 g |
| tryptophan | 0.06 g |
| cystine | 0.15 g |

### (C) Organic acid composition (per 100 g)

| | |
|---|---|
| citric acid | 0.81 g |
| malic acid | 0.31 g |
| succinic acid | 0.12 g |
| lactic acid | 1.17 g |
| formic acid | 0.03 g |
| pyruvic acid | 0.01 g |
| free γ-aminobutyric acid | 24 mg |

### (D) Mineral composition (per 100 g)

| | |
|---|---|
| phosphorus | 262 mg |
| iron | 2.65 mg |
| calcium | 72.1 mg |
| potassium | 798 mg |
| magnesium | 97.8 mg |
| zinc | 1.19 mg |
| iodine | 1.7 mg |

This plant fermented product is the same as Tensei Koso Kinjirushi (manufactured by NIHON SIZEN HAKKOH CO., LTD.), and this was used in the following Examples. When this was administered to humans, one in which a predetermined amount of the plant fermented product was enclosed in an aluminum sheet and stored at room temperature until use was used.

### Example 1

### Administration Test and Collection of Questionnaire

A hearing survey was conducted on purchasers who regularly purchase a plant fermented product (Tensei Koso Kinjirushi) and factory tour visitors, etc. to whom the plant fermented product (Tensei Koso Kinjirushi) produced in Production Example 1 was distributed (a total of about 3000 people) using an optional and voluntary questionnaire method (with permission to use the questionnaire). The answerers were asked to freely write subjective symptoms felt in the body and other experience information together with the ingestion status without providing preliminary information about the efficacy, and among the approximately 3100 answers, mere letters of appreciation were excluded, and answers describing some symptoms were extracted for each large category (emotions/behaviors, geriatric symptoms, digestive symptoms, chronic fatigue, chronic inflammation, metabolic diseases, etc.) associated with QOL (the extraction status of the questionnaire is shown in the following Table 1). The number of adopted answers was 1679, and multiple answers were used for classification according to symptoms, and the total number of effective cases sorted by total number of cases was 2268. Further, the ingestion amount (g/day) in Examples was estimated from the sales record or the distribution record of each person. Incidentally, % in the table indicates the improvement ratio.

Note that this test was not a randomized comparative test, and mainly included qualitative answers, but also included a description of changes in test data. It is a raw description of subjective and objective symptoms, and since the number of samples is large, the effect of the plant fermented product can be ascertained and is valuable data. The classification was performed according to the following criteria. The diagnostic criteria for chronic fatigue syndrome of the Ministry of Health, Labor and Welfare are also centered on the description of subjective symptoms, and the effect on objective symptoms of a cytokine or the like is not included in the criteria, and therefore, a comparative study will be difficult.

**[Table 1]**

| Total number of collected answers | Number of excluded cases* | Number of effective cases | | | | Number of cases where QOL was improved | | | |
|---|---|---|---|---|---|---|---|---|---|
| | | total number | male | female | unknown | total number | male | female | unknown |
| 3104 | 450 | 2493 | 674 | 1817 | 2 | 2268 | 620 | 1646 | 2 |

| | | | | | | | | | |
|---|---|---|---|---|---|---|---|---|---|
| * Symptoms are not described: A case where the effect cannot be evaluated such as a mere letter of appreciation, a case where there were no current symptoms, but the ingestion was performed while expecting to have a health effect, a case where the ingestion period was short, etc. were excluded. | | | | | | | | | |

From the above results, it was found that the plant fermented product (Tensei Koso Kinjirushi) produced in Production Example 1 has a QOL improving effect. Hereinafter, the QOL improving effect will be described in detail for each large category.

### <Emotions/Behaviors>

The subjective symptoms of "energetic" in "getting energetic by eating" in this large category is a subjective and autonomous response, and the corresponding answers to the questionnaire were diverse. When the answers were daringly classified, they were classified as shown in the following Table 2. The number of cases in each medium category are shown in Table 3 according to gender, improved, unchanged, and undeterminable (the effect cannot be determined because the ingestion period is short).

**[Table 2]**

| Medium category | Typical subjective or objective effect |
|---|---|
| Energetic/feeling/ physical condition | stay healthy, feel good, feel positive, reduce depressive symptoms |
| Eating behavior | have appetite and feel meal is delicious, low body weight was restored |

**[Table 3]**

| Medium category | Small category | male | female | ? | improved | unchanged | exacerbated | g/day | % |
|---|---|---|---|---|---|---|---|---|---|
| Energetic/ physical condition | Energetic/ sense of health | 78 | 158 | 0 | 236 | 0 | 0 | 9.81 | 61 |
| | Feeling | 14 | 60 | 0 | 74 | 0 | 0 | 7.98 | 19 |
| | Subjective/ objective | 11 | 17 | 0 | 28 | 0 | 0 | 5.92 | 7 |
| Eating behavior | Increased appetite | 13 | 37 | 0 | 50 | 0 | 0 | 17.91 | 13 |
| | Decreased appetite | 1 | 0 | 0 | 1 | 0 | 0 | 6.82 | 0 |
| Total | | 117 | 272 | 0 | 389 | 0 | 0 | | 100 |
| Average | | | | | | | | 9.69 | |

| | | | | | | | | | |
|---|---|---|---|---|---|---|---|---|---|
| ?: Gender not known | | | | | | | | | |

The phenomenon that "by eating the plant fermented product, somehow getting energetic", which is a previous voice of consumers, was ascertained as the number of cases. When those indicated by the ambiguous expression were classified according to symptoms, evaluations from a comprehensive and mentally subjective one to an objective one that a subject looks younger than age were observed. Depressive symptoms are objectively found in the overall impression. In a patient with chronic lymphoma and a patient with memory impairment, improvement of depressive symptoms has been observed objectively with this plant fermented product.

If energetic, physical condition, and sense of health are combined, answers regarding the improvement ratio account for 80% in this category, and not only subjective impression, but also objective comments from family and friends are obtained. Not only feeling, but also an appetite is given to a subject whose body weight is as low as 30 kg or more and less than 40 kg, and the low body weight has escaped from the dangerous range. There were no exacerbation cases.

### <Aging Symptoms>

The answers to questionnaire corresponding to the subjective symptoms in this large category were diverse. When the answers were daringly classified, they were classified as shown in the following Table 4. The number of cases in each medium category are shown in Table 5 according to gender, improved, unchanged, and undeterminable (the effect cannot be determined because the ingestion period is short).

**[Table 4]**

| Medium category | Typical subjective effect |
|---|---|
| Healing ability | enhance natural healing ability, recover faster after surgery and reduce dosage, improve test data |
| Appearance | looked younger, shininess and suppleness of skin, hair, etc. were pointed out |
| Function | active behavior, shininess of skin, suppression of curvature of spine, etc. |
| Physical strength/ motor ability | get physical strength, no fatigue during exercise or work, (associated with frailty) |

**[Table 5]**

| Medium category | Small category | male | female | ? | improved | unchanged | exacerbated | g/day | Improvement % |
|---|---|---|---|---|---|---|---|---|---|
| Healing ability | Natural healing ability | 6 | 19 | 0 | 25 | 0 | 0 | 6.82 | 11 |
| | Postoperative recovery | 16 | 27 | 0 | 43 | 0 | 0 | 6.20 | 20 |
| Appearance | Overall state | 0 | 2 | 0 | 2 | 0 | 0 | - | 1 |
| Function | Behavior/skin/eye s/spine | 25 | 44 | 0 | 69 | 0 | 0 | 6.11 | 31 |
| Physical strength/ motor ability | Frailty | 20 | 62 | 0 | 82 | 0 | 0 | 12.49 | 37 |
| Total | | 67 | 154 | 0 | 221 | 0 | 0 | | 100 |
| average | | | | | | | | 7.91 | |

| | | | | | | | | | |
|---|---|---|---|---|---|---|---|---|---|
| ?: Gender not known | | | | | | | | | |

Recovery ability decreases with aging. A subjective effect suggestive of the strengthening of the healing ability was observed. Further, answers that recovery after surgery for cancer or the like is fast, and the test data were improved, and the dosage was reduced are noteworthy. There was an answer that the shininess and suppleness of the skin, hair, facial expressions, etc. looked younger to others. This matches the effect of suppressing geriatric symptoms seen in SAM mice. It was pointed out that it contributes not only to exercise but also to farm work and transportation so that a subject can be aware of having gained physical strength. It seems that "frailty" (frail tendency), which has become a problem in an aging society, is improved. What is noteworthy is the description of the recovery ability after surgery. Most of the postoperative cases are gastric cancer, liver cancer, lung cancer, etc., but there were conspicuous answers that the postoperative recovery is fast or that good health can be perceived. Not only the sense of health, but also the objective effect is confirmed, for example, the test data was improved, or the reduction of a therapeutic agent was proposed by the doctor.

As aging progresses, the healing ability gradually decreases. In the long-term breeding test using SAM senescence-accelerated model mice, the ingestion of the plant fermented product showed inhibition of aging in all the items of the aging index specified by the Society for SAM Research, and also showed life extension. With the aging of the population, sarcopenia is increasing, and the number of cases requiring nursing care due to fall or the like is increasing. A method for preventing this has been required, and the Ministry of Health, Labor and Welfare also needs measures. There are some health foods for measures against locomotive syndrome, but most of them are branched amino acids, proteins, and a joint strengthening action, and it is noteworthy that the plant fermented product makes a subject perceive the frailty suppressing effect. In the large category "energetic/physical condition", the improvement ratio of "energetic/sense of health" is as high as 60%, and the mental aspect may contribute to the frailty improving effect. In the field of Kampo medicine, greater importance is attached to the improvement of healing ability, and also in this category, a mental action attracts attention. It seems that the healing ability perceived by the reduction of the dose of a medication as well as the improvement of the disease state or the test data is a new action. As for the contents of the function, the scores in the four items of aging evaluation of SAM mice: behaviors, skin symptoms, eye inflammation, and curvature of spine were employed. The improvement ratio was 50%, and it was shown that it is also applied to the anti-aging action in humans.

### <Digestive Symptoms>

In this large category, there were many comments on bowel movement (especially constipation). The proportion of the elderly is high in the consumers. This is because the digestive organ also ages due to aging. It has become clear that the digestive system is deeply related to immunity as well as food digestion and defecation functions, and is directly connected to the brain function. It is being recognized as an important category of QOL. The classification is as shown in the following Table 6. The number of cases in each medium category is shown in Table 7 according to gender, improved, unchanged, and undeterminable (the effect cannot be determined because the ingestion period is short).

**[Table 6]**

| Digestive symptoms | | Summary of answers |
|---|---|---|
| Bowel movement | Constipation | Many regular users due to improvement of constipation. Pharmaceutical laxative is too potent and inconvenient |
| | Diarrhea | Number of cases is small. Effective case also for ulcerative colitis. There is a case where mild diarrhea occurred when starting to drink |
| Stomach symptoms | | There are many cases of getting hungry. Upset stomach is eliminated. |

**[Table 7]**

| Medium category | Small category | male | female | ? | improved | unchanged | exacerbated | g/day | % |
|---|---|---|---|---|---|---|---|---|---|
| Bowel movement/ Stool properties | Constipation symptoms | 32 | 132 | 0 | 164 | 1 | 1 | 11.02 | 53 |
| | Diarrhea symptoms | 4 | 6 | 0 | 10 | 0 | 2 | 17.91 | 3 |
| | Stool properties | 9 | 11 | 0 | 20 | 0 | 0 | 23.68 | 7 |
| Other gastrointestinal symptoms | Pharyngeal symptoms | 0 | 1 | 0 | 1 | 0 | 0 | 6.47 | 0.0 |
| | Stomach symptoms | 15 | 33 | 0 | 48 | 0 | 1 | 10.44 | 16 |
| | Large intestine symptoms | 11 | 12 | 0 | 23 | 0 | 0 | 12.09 | 7 |
| | Rectum/anus | 15 | 25 | 1 | 41 | 0 | 0 | 4.09 | 13 |
| Total | | 86 | 220 | 1 | 307 | 1 | 5 | | 100 |
| Average | | | | | | | | 12.24 | |

| | | | | | | | | | |
|---|---|---|---|---|---|---|---|---|---|
| ?: Gender not known | | | | | | | | | |

Most of the QOL improving effects in this category were improvement of constipation. Constipation has come to be recognized in Japan as an important item of QOL. The number of cases revealed that the constipation eliminating effect is strongly desired by the consumers. In the comments on the stomach symptoms, comments that "appetite is increased", "the stomach feels refreshed" were seen, and it was found that the stomach symptoms are diverse. For those with a weak constitution, it leads to body weight gain and seems to have a QOL improving action. Although the number of cases for diarrhea symptoms is small, attention is paid to the answer regarding the effect on ulcerative colitis, for which there are few effective therapeutic methods.

Bowel movement is recognized as an important category of QOL such as discomfort and limitation of range of action. In the answers to the questionnaire, the frequency of constipation improvement was high. Constipation and diarrhea are deeply related to the central nervous system and the state of enteric bacteria in the activity of the digestive system, and are being recognized as QOL. In bowel movement dysfunction, constipation and diarrhea are in the lower gastrointestinal tract, but symptoms such as "stomach symptoms in which stomach is upset" and mental symptoms such as a symptom in which appetite is increased were classified. Reflux of gastric juice, stomach pain, etc. were divided in chronic inflammation category.
1) The most common answer regarding improvement of bowel movement is a constipation improving effect. In particular, there were many improvement cases in females.
2) In Japan, the ingestion of dietary fibers such as vegetables is recommended as a treatment for constipation, and it seems that QOL is not given so much importance as in Europe and the United States. In Europe and the United States, constipation is regarded as an important factor for QOL, and scientific research is also actively conducted.
3) The ratio of the bowel movement improving effect of the applied plant fermented product reached 166 cases in the number of cases to be evaluated, and the reproducibility of the subjective effect is also high. When a regular user (5 g/day) who could not expect the effect of a medicine due to constipation resumed ingestion in the case of discontinuation of administration for 3 weeks, recovery was achieved in 3 days. The amount of dietary fibers (enzyme weight method) of this plant fermented product is about 5 g/100 g, which is only a medium amount such as green soybeans. In particular, the elderly people are more likely to have constipation due to intestinal aging. A constipation improving agent is prescribed, but the disadvantage is that it is not possible to predict when to have a bowel movement, and in particular, it is difficult to find a rest room while traveling. The plant fermented product have a mild effect, and therefore is appreciated. In terms of stool properties, there was a comment that the pleasant sensation of passing a banana-like stool smoothly is exactly the improvement of QOL.
4) Although the effect on stomatitis is also pointed out, it was classified as chronic inflammation.

### <Chronic Fatigue>

As the targets in this large category, categories, such as mental fatigue, muscle fatigue, and metabolic fatigue of general fatigue such as hardly get tired and recovered from fatigue, become targets. When these were daringly classified, these were classified as shown in the following Table 8. The number of cases in each medium category are shown in Table 9 according to gender, improved, unchanged, and undeterminable (the effect cannot be determined because the ingestion period is short). Complaints about chronic fatigue tend to increase and chronic fatigue becomes an important target for improving QOL. The mechanism is becoming clear, and cases where the therapeutic effect is aimed at pinpoint are also increasing. There is chronic inflammation in the basis, but chronic fatigue is mainly a subjective evaluation such as a bodily sensation, and it is counted separately from the chronic inflammation whose cause is easy to identify.

**[Table 8]**

| Medium category | Typical subjective effect |
|---|---|
| Skin/hair | Evaluated as follows: shininess and suppleness of skin look younger than age. Important in terms of QOL. |
| | Also for SAM mice, aging was evaluated based on fur and shininess of fur. The same shall apply to internal organs. |
| Digestive system | Stomach symptoms: mental symptoms other than gastritis such as somehow heavy on stomach or no appetite |
| Sleep | sleep quantity and quality: ease of falling asleep, refreshed feeling upon awakening, sense of lack of sleep |
| Sense of fatigue | Improvement of somehow tired or lack of motivation although no clear cause can be identified. Mainly muscle fatigue, but regarded important for those with a weak constitution. classified into geriatric symptoms |
| Autonomic nerve control | Heart pounding and dizziness due to blood pressure fluctuations, cold constitution, sweating, sense of fever of body, etc. |
| Microcirculatory system | Cold constitution or stiff shoulder |
| Immune fatigue | Infection is a cause of chronic fatigue, easy to catch cold, effect on warts |

**[Table 9]**

| Medium category | Small category | male | female | ? | improved | unchanged | exacerbated | g/day | % |
|---|---|---|---|---|---|---|---|---|---|
| Skin/hair | Hair condition | 25 | 47 | 0 | 72 | 0 | 0 | 8.23 | 10 |
| | Skin condition | 35 | 100 | 0 | 135 | 0 | 0 | 5.02 | 18 |
| Digestive system | Gastrointestinal symptoms | 1 | 0 | 0 | 1 | 0 | 0 | - | 0.0 |
| Sleep | Good sleep/wake | 37 | 113 | 0 | 150 | 0 | 1 | 12.82 | 20 |
| | Lack of sleep | 4 | 5 | 0 | 9 | 0 | 0 | 6.63 | 1 |
| Sense of fatigue | Sense of mental fatigue | 32 | 135 | 0 | 167 | 0 | 0 | 12.69 | 22 |
| | Sense of physical fatigue | 9 | 22 | 0 | 31 | 0 | 0 | 5.24 | 4 |
| Autonomic nervous system | Blood pressure fluctuations | 0 | 1 | 0 | 1 | 0 | 0 | - | 0.0 |
| | Sweating | 5 | 12 | 0 | 17 | 0 | 0 | 6.92 | 2 |
| | Body temperature | 3 | 22 | 1 | 26 | 0 | 0 | 3.86 | 3 |
| | Dizziness | 2 | 11 | 0 | 13 | 0 | 0 | 7.57 | 2 |
| | Motion sickness | 1 | 13 | 0 | 14 | 0 | 0 | 16.96 | 2 |
| Microcirculatory system | Cold constitution | 0 | 29 | 0 | 29 | 0 | 0 | 6.73 | 4 |
| | Stiff shoulder | 2 | 39 | 0 | 41 | 0 | 0 | 4.85 | 5 |
| Immune fatigue | Immune fatigue | 11 | 29 | 0 | 40 | 0 | 0 | 9.12 | 5 |
| Total | | 167 | 578 | 1 | 746 | 0 | 1 | | 100 |
| Average | | | | | | | | 8.20 | |

| | | | | | | | | | |
|---|---|---|---|---|---|---|---|---|---|
| ?: Gender not known | | | | | | | | | |

Chronic fatigue is a very difficult category, but there is a great need to improve indefinite complaints. In fact, there were answers in a wide range of categories. The expression "get less tired by eating" was also seen. The description of suppleness and shininess of the skin was from both aspects of subjective and objective symptoms. It has been known that in addition to physical fatigue, the sense of fatigue in which the central nervous system is involved and the autonomic nerve is involved in the sense of fatigue. It is difficult to detect the activity of the autonomic nerve, and therefore, classification was attempted according to symptoms in the answers. As the skin symptoms, the skin is shine and supple, and the expression of "look younger" was seen. A state of being exhausted in the overall impression also falls under this category. In the category of exercise/muscle fatigue, as for the expression of fatigue, the expressions that "get less tired even after exercising and that gained physical strength were individually classified. The stomach symptoms were such that a subject had no appetite for some reason. However, it seems to be different from simple appetite. As for sleep, many suffer from insomnia. Quantity and quality are issues, and it is not that REM sleep and non-REM sleep are measured in terms of expression. Rather, the expression of QOL improvement was such that a subject slept well and felt refreshed upon awakening. The reason is not clear, but there is a sense of fatigue. This indicates a case where it may be caused by a metabolic wasting disease, but the cause cannot be identified. It has recently been elucidated that autonomic nerve control is one of the critical causes of sense of fatigue. Although it is not possible to measure the activity factor of autonomic nerve by oneself without a special instrument, not the blood pressure value, but heart pounding or dizziness due to blood pressure fluctuations, cold constitution, sweating, sense of fever of the body, etc. were classified into this category. It is a category where QOL improvement can be felt in the body when it is improved.

### <Chronic Inflammation>

An acute inflammatory response is a series of biological responses of repair actions for urgent conditions such as wounds or infections, and after a series of responses such as recognition of a dysfunction, pain, and redness are completed, a series of termination responses toward healing occur. This is a normal biological response. On the other hand, in the process of terminating acute inflammation, it is often the case that the inflammatory response is weak but the inflammatory state continues, which becomes chronic inflammation and is the basis of many chronic diseases. It has become clear that this chronic inflammation exists as the basis of various dysfunctions in the living body, for example, lifestyle-related diseases such as so-called diabetes mellitus, obesity, cancer (development/metastasis, etc.), aging, autoimmunity, etc. For example, it has become clear that some kind of stress causes inflammation in microglial cells in the central nervous system, and an inflammatory factor is carried throughout the body through the bloodstream, and triggers other inflammatory symptoms. It is estimated that this may happen in the early stage of dementia, and the search for a new therapeutic or preventive agent has begun. So-called allergies and inflammation of various organs are classified in this category. Chronic inflammation is important as an alarm function, and there are many recognizable symptoms such as pain and redness. The contents of answers were also a wide range of symptoms. The answers to questionnaire corresponding to subjective symptoms in this large category were diverse. When the answers were daringly classified, they were classified as shown in the following Table 10. The number of cases in each medium category are shown in Table 11 according to gender, improved, unchanged, and undeterminable (the effect cannot be determined because the ingestion period is short). Note that obesity was classified into this category because it is inflammation of adipocytes, and insulin resistance and diabetes mellitus are chronic inflammation. In addition, mere body weight gain and loss are also classified into this category.

**[Table 10]**

| Medium category | Summary of answers |
|---|---|
| Digestive inflammation | Stomatitis heals faster. There are also effective cases for stomach pain, reflux gastritis, and ulcerative colitis. also effective for hemorrhoid |
| Liver inflammation | liver function/enlarged liver, GOT/GPT test data were high. hepatitis C |
| Lung inflammation | There are cases related to sore throat, bronchitis, pneumonia, and asthma. |
| Genitourinary tract inflammation | cystitis, urethritis, nephritis, prostatitis, physiology |
| Skin inflammation | effective for redness and itching by direct application, rough skin |
| Circulatory inflammation | varicose vein, cerebral thrombosis, cerebral infarction, etc. Blood pressure value is classified into other QOL category. Cold constitution is poor peripheral circulation. |
| Brain inflammation | encephalitis, meningitis, depression (if diagnosed), related to memory and cognition, cerebral symptoms due to cerebrovascular disorder, Dementia is difficult to diagnose, and therefore evaluated based on symptoms, mainly objective symptoms |
| Joint inflammation | pain in hands, elbows, knees, lower back, etc. (vertebral hernia, stenosis, etc.) |
| Muscle inflammation | muscle pain, stiff back, etc. |
| Nerve inflammation | neuritis, neuralgia, numbness in limbs, also headache, etc. |
| Allergy | Allergy in general is extracted, atopy, no food allergy |
| Sensory inflammation | tinnitus increases with aging, but the cause is unknown, thickening of retina was improved |
| Tumor (metastasis/recurrence) | Inflammation is involved in the development, growth, metastasis, and recurrence of cancer. Recovery after surgery is classified into healing ability. |
| Infection | tinea pedis, warts, etc. |
| Pain in general | pain in roughly categorized body parts (legs, body) |
| Other inflammation | rheumatism, empyema, Behcet's disease |

**[Table 11]**

| Medium category | Small category | male | female | ? | improved | unchanged | exacerbate d | g/day | % |
|---|---|---|---|---|---|---|---|---|---|
| Digestive inflammation | Stomatitis, tonsillitis | 1 | 23 | 0 | 24 | 0 | 0 | 7.86 | 3 |
| | Gastritis | 6 | 11 | 0 | 17 | 0 | 0 | 5.67 | 2 |
| | Enteritis | 1 | 2 | 0 | 3 | 0 | 0 | 10.39 | 0.0 |
| | Rectum/anus | 0 | 2 | 0 | 3 | 0 | 0 | 10.39 | 0.0 |
| Liver inflammation | Liver function/enlarged liver | 14 | 13 | 0 | 27 | 0 | 0 | 7.33 | 4 |
| | Hepatitis C | 1 | 5 | 0 | 6 | 0 | 0 | 26.61 | 1 |
| Lung inflammation | Bronchitis/pneumonia | 4 | 2 | 0 | 6 | 0 | 0 | 6.65 | 1 |
| | Others | 1 | 1 | 0 | 2 | 0 | 0 | 45.58 | 0.0 |
| Genitourinary tract inflammation | Cystitis, urethritis | 0 | 4 | 0 | 4 | 0 | 0 | 2.80 | 0.0 |
| | Nephritis | 2 | 0 | 0 | 2 | 0 | 0 | - | |
| | Genital tract inflammation | 2 | 5 | 0 | 7 | 0 | 0 | 10.26 | 1 |
| Skin inflammation | Dermatitis | 4 | 18 | 0 | 22 | 0 | 2 | 14.74 | 3 |
| Circulatory inflammation | Blood vessel | 2 | 7 | 0 | 9 | 0 | 0 | 3.03 | 1 |
| | Cerebral blood vessel | 6 | 3 | 0 | 9 | 0 | 0 | 10.90 | 1 |
| Brain inflammation | Encephalitis, meningitis | 1 | 0 | 0 | 1 | 0 | 0 | - | |
| | Depressive illness | 2 | 4 | 0 | 6 | 0 | 0 | 6.83 | 1 |
| | Memory, cognition | 1 | 1 | 0 | 2 | 0 | 0 | 5.00 | 0.0 |
| Joint inflammation | Spine, lower back | 1 | 5 | 0 | 6 | 0 | 0 | 22.07 | 1 |
| | Fingers | 5 | 25 | 0 | 30 | 0 | 0 | 30.50 | 4 |
| Muscle inflammation | According to individual parts | 1 | 4 | 0 | 5 | 0 | 0 | 6.19 | 1 |
| Nerve inflammation | Neuritis, neuralgia | 12 | 54 | 0 | 66 | 0 | 0 | 14.16 | 9 |
| Allergy | Organ allergy | 6 | 24 | 0 | 30 | 0 | 0 | 6.40 | 4 |
| | Food allergy | 3 | 6 | 0 | 9 | 0 | 0 | 19.34 | 1 |
| Sensory inflammation | Ear | 3 | 15 | 0 | 18 | 2 | 0 | 3.40 | 2 |
| | Eye | 4 | 3 | 0 | 7 | 0 | 0 | 19.48 | 1 |
| | Others | 0 | 1 | 0 | 1 | 0 | 0 | - | 0.0 |
| Tumor metastasis/ recurrence | According to individual organs | 11 | 16 | 0 | 27 | 0 | 0 | 5.24 | 4 |
| | Blood system | 1 | 0 | 0 | 1 | 0 | 0 | - | 0.0 |
| Infection | According to individual parts | 4 | 13 | 0 | 17 | 0 | 0 | 5.40 | 2 |
| Pain in general | According to individual parts | 1 | 4 | 0 | 5 | 2 | 0 | 1.42 | 0.0 |
| Other inflammation | Other inflammation | 2 | 1 | 0 | 3 | 0 | 0 | 21.08 | 0.0 |
| Total | 754 | 102 | 273 | 0 | 375 | 2 | 2 | | 100 |
| Average | | | | | | | | 12.23 | |

| | | | | | | | | | |
|---|---|---|---|---|---|---|---|---|---|
| ?: Gender not known | | | | | | | | | |

There was an answer that inflammation was relieved by direct application to the inflamed part of the skin. Many answers that stomatitis was relieved were seen. There were answers that inflammatory responses such as stomach pain, heartburn, reflux gastritis, and upset stomach are relieved. In the liver function, the test data of GOT and GPT decreased, and there was also an effect on fatty liver. The body weight gain and loss related to fatty liver are also summarized in this category. Nephritis was regarded as a concomitant symptom in the exacerbation of diabetes mellitus, and cystitis was also included in this category. Although there were many answers regarding blood pressure in the circulatory system, blood pressure fluctuations were not included in this category because they are derived from various regulatory mechanisms, and were counted in "other QOL-related category". A hyperlipidemia/thrombosis-related category was adopted. Diabetes mellitus is based on chronic inflammation, and as the disease state progresses, it leads to systemic inflammation such as nephritis and vasculitis, and therefore is summarized in this category. Since chronic inflammation is involved in carcinogenesis, growth, metastasis, recurrence, etc. of cancer on the whole, it was set as a medium category. There were conspicuous answers that the plant fermented product gives energy after cancer surgery, which was classified not into this category, but into "medium category: healing ability" in the large category "energetic". There was also an answer that malignant lymphoma was cured, but it was classified according to the answer although there is also a possibility of the effect of drug treatment. It is not easy to confirm the effect of suppressing metastasis/recurrence, and it has not been confirmed. Bronchial asthma, cough, etc. were occasionally found in the respiratory category and allergy. There was an answer that the symptoms of chronic rheumatoid arthritis, cataract, and glaucoma were improved a little. There were also answers that maxillary empyema was cured, and that the head after surgery for maxillary empyema felt heavy, but the body felt lighter and the work can be continued.

### <Metabolic Diseases, etc.>

The physiological responses associated with QOL improvement are summarized in this category. The answers to questionnaire corresponding thereto were diverse. When the answers were daringly classified, they were classified as shown in the following Table 12. The number of cases in each medium category are shown in Table 13 according to gender, improved, unchanged, and undeterminable (the effect cannot be determined because the ingestion period is short). Note that diabetes mellitus, hyperlipidemia, and obesity are accompanied by chronic inflammation as an underlying disease, but are classified as metabolic diseases here.

**[Table 12]**

| Medium category | Summary of answers |
|---|---|
| Circulatory system (blood pressure) | Improvement of blood pressure value was observed. |
| Diabetes mellitus | Answer of decrease in blood glucose level and accompanying improvement of blood glucose level and decrease in Hba1c |
| Hyperlipidemia | Decrease in cholesterol and neutral fat |
| Obesity | Reduction in excess body weight, recovery from low body weight |
| Other metabolic diseases | Changes in clinical test data, etc. Biochemical test data (uric acid, etc.), cholesterol is classified into "hyperlipidemia". |
| Climacteric, etc. | There were climacteric symptoms and physiology-related cases, which are not a disease but are treated as metabolic-related cases |

**[Table 13]**

| Medium category | Small category | male | female | ? | improved | unchanged | exacerbated | g/day | % |
|---|---|---|---|---|---|---|---|---|---|
| Circulatory system | Blood pressure | 27 | 56 | 0 | 83 | 1 | 0 | 7.11 | 36 |
| | Heart | 0 | 6 | 0 | 6 | 0 | 0 | 13.53 | 3 |
| Diabetes mellitus | Blood glucose level, Hba1c | 26 | 31 | 0 | 57 | 4 | 0 | 7.19 | 25 |
| | Concomitant symptoms | 3 | 0 | 0 | 3 | 0 | 0 | 10.00 | 1 |
| Hyperlipidemia | Cholesterol | 8 | 16 | 0 | 24 | 0 | 1 | 9.47 | 10 |
| | Neutral fat | 5 | 11 | 0 | 16 | 0 | 0 | 16.33 | 7 |
| Obesity | Excess body weight | 7 | 13 | 0 | 20 | 1 | 0 | 3.93 | 9 |
| | Low body weight | 3 | 13 | 0 | 13 | 0 | 0 | 31.29 | 6 |
| Other metabolic diseases | Clinical test data, etc. | 1 | 5 | 0 | 6 | 0 | 0 | 7.25 | 3 |
| Climacteric, etc. | Climacteric symptoms, physiology | 0 | 1 | 0 | 1 | 0 | 0 | 3.28 | 0.0 |
| Total | | 80 | 149 | 0 | 229 | 6 | 1 | | 100 |
| Average | | | | | | | | 10.93 | |

| | | | | | | | | | |
|---|---|---|---|---|---|---|---|---|---|
| ?: Gender not known | | | | | | | | | |

Improvement in blood pressure was seen in many cases. It is interesting that not only the blood pressure is normalized, but also there is a case where the dose of an antihypertensive agent that has been taken is reduced or an antihypertensive agent is no longer needed. The blood pressure is the result of cardiac discharge amount x vascular resistance, but the factors that affect this are diverse. Since many of the answerers to the questionnaire are elderly people, there are various factors such as a decrease in flexibility of peripheral blood vessels and a mental effect.

### <Reduction of Dose or Termination of Medication>

Among the answers to the questionnaire, cases leading to reduction of the dose or termination of a medication in use were sometimes seen in various categories, and therefore, such cases were extracted as a list. As shown in Table 14, it covers a wide range of categories, but among them, there was also a case where it was removed from the prescription at doctor's discretion not at the discretion of the answerer. The efficacy as an auxiliary effect in the treatment was also extrapolated.

**[Table 14]**

| Large category | Medium category | Small category | Reduction of dose of medication | Termination of medication |
|---|---|---|---|---|
| Emotions/ behaviors | Energetic/ physical condition | Energetic/ sense of health | | 3 |
| | | feeling | 1 | |
| | Healing ability | Natural healing ability | | 1 |
| | | postoperative recovery ability | 4 | |
| Geriatric symptoms | Function | eye | | 1 |
| Digestive symptoms | Bowel movement! stool properties | Constipation symptoms | | 3 |
| Chronic fatigue | Sleep | insomnia | 1 | 1 |
| | Immunity | immune fatigue | 1 | 3 |
| Chronic inflammation | Digestive inflammation | gastritis | | 1 |
| | Tumor | gastric cancer | 1 | |
| | Genitourinary tract system | prostatic hyperplasia | | 1 |
| | Skin | dermatitis | | 1 |
| | | rash | | 1 |
| | | tinea pedis | | 1 |
| | Brain | depressive illness | 1 | |
| | Joint | knee joint osteoarthritis | | 1 |
| | Nerve | headache | | 3 |
| | Allergy | urticaria | 1 | |
| | | pollinosis | | 2 |
| | | atopy | | 1 |
| Metabolic diseases, etc. | Circulatory system | blood pressure | 5 | 4 |
| | Diabetes mellitus | hyperglycemia | | 1 |

### <Improvement of Pain>

Since the questionnaire was classified from the viewpoint of OQL based on the pathological condition, a tendency that "improvement of pain" is dispersed in many categories was observed. Therefore, extraction from the results of the questionnaire was attempted with the keyword "pain", which is shown in Table 15.

**[Table 15]**

| Part/type | Number of cases | Summary of improved pain | % |
|---|---|---|---|
| Nerve pain | 2 | Improvement of sciatic neuralgia, trigeminal neuralgia | 1.7 |
| Rheumatic pain | 1 | Rheumatoid pain relief, and 3 other successful cases of rheumatoid arthritis (no mention of pain) | 1.5 |
| Knee pain | 30 | Improvement of knee pain alone and knee pain and lower back pain -> easier to ascend and descend stairs, revitalized as a whole | 26.1 |
| Lower back pain | 12 | Most lower back pain involves spine, muscular pain is counted separately | 10.4 |
| Spinal pain | 5 | relief of pain involving entire spine, cervical spine pain is also included | 4.3 |
| Shoulder pain | 2 | Similar to frozen shoulder. Pain is relieved. | 1.7 |
| Muscular pain | 8 | Pain considered to be muscular pain is relieved. | 7.0 |
| Headache | 24 | There are various causes of headache, but summarized as headache -> relieved | 20.9 |
| Gastrointestinal pain | 8 | improvement or elimination of stomach pain (including stomatitis pain and tongue pain) | 7.0 |
| Other unclassifiable pain | 13 | relief of unclassifiable pain such as varicose vein pain, tonsil pain, and menstrual pain | 11.3 |
| subtotal | 105 | - | 91.3 |
| Unevaluable, etc. subtotal | 10 | - | 8.7 |
| Total pain-related cases | 115 | - | 100.0 |

There were 115 answers with the keyword "pain", and 10 of them were determined to be "unevaluable" because a sufficient time has not elapsed since the plant fermented product started to be ingested and evaluation was not performed. The number of answers in which some effectiveness was perceived was 105, and the categories were diverse. There were no exacerbation cases. In this manner, the effect of improving pain was also confirmed.

### <Summary>

From the above results, it was confirmed that the plant fermented product improves QOL based on large categories (emotions/behaviors, geriatric symptoms, digestive symptoms, chronic fatigue, chronic inflammation, metabolic diseases, etc.). A summary of these is shown in Table 16.

**[Table 16]**

| Large category | Medium category | Small category | Improved | | | | Unchanged | Exacerbated | Total | Improvement % |
|---|---|---|---|---|---|---|---|---|---|---|
| | | Contents | Male | Female | ? | Total | | | | |
| Emotions/ Behaviors | Energetic/ physical condition | Sense of energy/sense of health/feeling/ subjective/ objective | 103 | 235 | 0 | 338 | 0 | 0 | 338 | 148 |
| | Eating behavior | Increased/ decreased appetite | 14 | 37 | 0 | 51 | 0 | 0 | 51 | 2.2 |
| (Subtotal) | | | 117 | 272 | 0 | 389 | 0 | 0 | 389 | 170 |
| Geriatric symptoms | Healing ability | Natural healing ability, postoperative recovery | 22 | 47 | 0 | 68 | 0 | 0 | 68 | 3.0 |
| | Appearance | Overall state | 0 | 2 | 0 | 2 | 0 | 0 | 2 | 0.0 |
| | Function | Behavior/ skin/eyes/ spine | 25 | 44 | 0 | 69 | 0 | 0 | 69 | 3.0 |
| | Physical strength/ motor ability | Frailty | 20 | 62 | 0 | 82 | 0 | 0 | 82 | 36 |
| (Subtotal) | | | 67 | 155 | 0 | 222 | 0 | 0 | 221 | 9.7 |
| Digestive symptoms | Bowel movement/ Stool properties | Constipation/ Diarrhea symptoms/ Stool properties | 45 | 149 | 0 | 194 | 1 | 3 | 198 | 8.5 |
| | Others | Pharynx, stomach, large intestine, rectum | 41 | 70 | 1 | 112 | 0 | 1 | 113 | 49 |
| (subtotal) | | | 86 | 219 | 1 | 306 | 1 | 4 | 311 | 13.4 |
| Chronic fatigue | Skin/hair | Skin/hair condition | 60 | 147 | 0 | 207 | 0 | 0 | 207 | 9.1 |
| | Digestive system | Gastrointestinal symptoms | 1 | 0 | 0 | 1 | 0 | 0 | 1 | 0.0 |
| | Sleep | Good sleep/wake, Lack of sleep | 41 | 118 | 0 | 159 | 0 | 1 | 160 | 70 |
| | Sense of fatigue | Sense of mental/ physical fatigue | 41 | 157 | 0 | 198 | 0 | 0 | 198 | 8.7 |
| | Autonomic nerve control | Blood pressure fluctuations, sweating, body temperature, dizziness, motion sickness | 11 | 59 | 1 | 71 | 0 | 0 | 71 | 31 |
| | Microcirculatory system | Cold constitution, stiff shoulder | 2 | 68 | 0 | 70 | 0 | 0 | 70 | 31 |
| | Immunity | Immune fatigue | 11 | 29 | 0 | 40 | 0 | 0 | 40 | 18 |
| (subtotal) | | | 167 | 578 | 1 | 746 | 0 | 1 | 747 | 32.6 |
| Chronic inflammation | Digestive inflammation | Stomatitis, tonsillitis, gastritis, proctitis | 8 | 38 | 0 | 46 | 0 | 0 | 46 | 20 |
| | Liver inflammation | Enlarged liver, hepatitis C | 15 | 18 | 0 | 33 | 0 | 0 | 33 | 1.4 |
| | Lung inflammation | Bronchitis, pneumonia, others | 5 | 3 | 0 | 8 | 0 | 0 | 8 | 0.4 |
| | Genitourinary tract inflammation | Cystitis, urethritis, nephritis, genital tract inflammation | 4 | 9 | 0 | 13 | 0 | 0 | 13 | 0.6 |
| | Skin inflammation | Dermatitis | 4 | 18 | 0 | 22 | 0 | 2 | 24 | 0.6 |
| | Circulatory inflammation | Vascular, cerebrovascular inflammation | 8 | 10 | 0 | 18 | 0 | 0 | 18 | 0.8 |
| | Brain inflammation | Encephalitis, meningitis, depressive illness, memory, cognition | 4 | 5 | 0 | 9 | 0 | 0 | 9 | 0.4 |
| | Joint inflammation | Spine, lower back, fingers | 6 | 30 | 0 | 36 | 0 | 0 | 36 | 16 |
| | Muscle inflammation | According to individual parts | 1 | 4 | 0 | 5 | 0 | 0 | 5 | 0.0 |
| | Nerve inflammation | Neuritis, neuralgia | 12 | 54 | 0 | 66 | 0 | 0 | 66 | 29 |
| | Allergy | Organ, food allergy | 9 | 30 | 0 | 39 | 0 | 0 | 39 | 16 |
| | Sensory inflammation | Ear, eye, others | 7 | 19 | 0 | 26 | 0 | 0 | 26 | 11 |
| | Tumor metastasis/ recurrence | According to individual organs, blood system | 12 | 16 | 0 | 28 | 0 | 0 | 28 | 10 |
| | Infection | According to individual parts | 4 | 13 | 0 | 17 | 0 | 0 | 17 | 0.7 |
| | Pain in general | According to individual parts | 1 | 4 | 0 | 5 | 2 | 0 | 7 | 0.0 |
| | Other inflammation | Other inflammation | 2 | 1 | 0 | 3 | 2 | 0 | 3 | 0.0 |
| (subtotal) | | | 102 | 272 | 0 | 374 | 4 | 0 | 378 | 16.4 |
| Metabolic diseases, etc. | Circulatory system | Blood pressure, heart | 27 | 62 | 0 | 89 | 1 | 0 | 90 | 3.3 |
| | Diabetes mellitus | Blood glucose level, concomitant symptoms | 29 | 31 | 0 | 60 | 4 | 0 | 64 | 39 |
| | Hyperlipidemia | Cholesterol, neutral fat | 13 | 27 | 0 | 40 | 0 | 1 | 41 | 18 |
| | Obesity | Excess body weight, low body weight | 10 | 26 | 0 | 36 | 1 | 0 | 37 | 16 |
| | Other metabolic diseases | Clinical laboratory test values | 1 | 5 | 0 | 6 | 0 | 0 | 6 | 0.3 |
| | Climacteric, etc. | Climacteric symptoms, physiology | 0 | 1 | 0 | 1 | 0 | 0 | 1 | 0.0 |
| (subtotal) | | | 80 | 152 | 0 | 232 | 6 | 1 | 239 | 10.1 |
| Total | | | 619 | 1648 | 2 | 2269 | 11 | 6 | 2285 | 1000 |

| | | | | | | | | | | |
|---|---|---|---|---|---|---|---|---|---|---|
| ?: Gender not known | | | | | | | | | | |

A noteworthy category in the list of the total number of cases is the medium category "Energetic/sense of health/ feeling", in which the total number of improved cases was 389, accounting for 14.9% of the total number of improved cases. In the QOL category, there are various categories, but 14.9% of the number of improved cases is a large number. In this category and the number of cases with improved healing ability (68 cases = 3.0%), it seemed that there was an improvement in "Energetic/sense of health/feeling" at the root. In modern Western medicine, such a concept is not major, but it is taken seriously in Kampo medicine and is classified as "superior (jo) medicine". In Western medicine, the cause of disease (diagnosis) and the treatment are required to have a clear 1:1 relationship. A treatment for an infectious disease with an antibiotic is a typical example. However, the antibiotic merely acts on a pathogenic microorganism and reduces its number. Since the number of pathogenic bacteria has decreased, it has only been cured by biological defense such as immunity. Abnormal bowel movement was not previously considered to be a category of QOL, but it has occupied an important position in QOL in Europe and the United States. The number of improved cases is large for both men and women and 194 cases, and the improvement ratio reaches 8.6%. It was perceived that this plant fermented product has an improving effect in a wide range of categories, and there are also some cases where also doctors propose to reduce the dose of a medication. Such a quality-of-life improving agent is novel.

### Example 2

Support for QOL improvement based on chronic inflammation:

### (1) Mice

SAM-P1 (female) mice at 8 weeks of age were purchased from Sankyo Lab Corporation, Inc. and used in each group with 6 mice. For breeding, a mouse breeding facility that complies with the SPF specification (23 ± 2°C, humidity: 50 ± 10%, lighting, light-dark cycle: 20:00 to 8:00) was used, and mice were bred individually under SPF conditions with a polycarbonate cage manufactured by CLEA Japan, Inc. using 6 mice per group for each sample. As the feed, 500N (γ-ray sterilized) purchased from Sankyo Lab Corporation, Inc. was ingested ad libitum. As the floor mat, one sterilized at high temperature manufactured by CLEA Japan, Inc. was used. The cage was changed once a week. Note that during the breeding, the feed ingestion amount of the mice did not decrease.

### (2) Sample

The plant fermented product produced in Production Example 1 was used as a sample. The plant fermented product was dissolved in ion exchanged water to a concentration of 2.0% or 0% (control), and dispensed in 500 ml-volume medium bottles, all of which were sterilized with an autoclave (121°C, 20 minutes), and the supernatant was aseptically transferred to a water bottle (sterilized) and used so that the nozzle of the water bottle would not be clogged with the formed precipitate. Note that the dose of the plant fermented product is a value calculated by referring to the case where a large amount of the plant fermented product is ingested on a daily basis (77 g/week = 11 g/day) and converting the amount equivalent to 30 times thereof to a value per Kg of body weight. This conversion ratio is a numerical value empirically used when applying Kampo medicines to mice at the Institute of Natural Medicine University of Toyama (Kinzo Matsumoto, Personal Information 28th SAM Study Group "Improving effect of Kampo medicine, Chotosan and Chuyaku, Kangen granules on cognitive behavioral disorders of aged model animal SAMP8 and their neural mechanisms", 5 July (2013) Aichi Gakuin University). When a mouse drinks about 30 ml of the sample at a concentration of 2.0% in one day, it corresponds to about 10 g/day in terms of human dose. The plant fermented product diluted with ion exchanged water as described above was ingested ad libitum as drinking water.

### (3) Collection of specimens

SAMP-1 (male) mice fed with the above sample as drinking water were bred for a long period of time up to 10 and 56 weeks of age in each group with 6 mice (test groups are shown below) . Each mouse was anesthetized with somnopentyl, followed by laparotomy, and then, the thymus and kidneys were collected, immersed in an ice-cooled RNA Later solution, and transported under refrigeration to a measuring institution (Filgen, Inc.). The RNA extraction and the measurement using DNA microarrays were also performed by the measuring institution.

**[Table 17]**

| Sample concentration | 0% | 0% | 2% |
|---|---|---|---|
| Breeding age | 10 weeks of age | 56 weeks of age | 56 weeks of age |

### (4) DNA microarray

The following gene-related expressions were analyzed from the analytical data (the number of genes: 41,354) obtained from the above institution.

### (a) Inflammation (Inflam)-related genes

When the analytical data obtained from the above institution was filtered by the keyword "Inflammation (inflame)", the number of cases was narrowed down to 566. In order to narrow down the genes with clear expression, the number of genes narrowed down by setting the increased ratio to 1.5 times or more and the decreased ratio to 0.66 times or less was 68. Clustering analysis was performed using these genes. The test groups compared in the clustering and the purposes are as follows. In addition, in DAN microarray analysis of the kidney, it was overwhelmingly less than that of the thymus, and the degree of increase or decrease was also small. In the explanation of each strain of the Society for SAM, it is stated that SAM-P1 is more likely to develop renal atrophy. However, there were no tissue abnormalities in the capillaries and renal pelvis in the kidneys collected in this test, and they need not be subjected to inflammation evaluation.

**[Table 18]**

| Comparison groups | (1) 0% 56 weeks of age | (2) 2% 56 weeks of age | (3) 2% 56 weeks of age |
|---|---|---|---|
| | vs | vs | vs |
| | 0% 10 weeks of age | 0% 10 weeks of age | 0% 56 weeks of age |
| Purpose of comparison | Phenomenon that occurs at old age | Effect of 2% ingestion at old age | Comparison between old age groups |

When examining the contents of Gene Description of 68 genes, it is shown that the inflammatory response in this test system can be analyzed with little bias because genes of major inflammation-related mediators, receptors, enzymes, and the like are included.

When a heat map reflecting the degree of increase or decrease in expression was created, the effect on old age at 0% of the sample was such that the degree of aging progressed (Patent: Anti-Aging Agent) and also the degree of inflammation increased, but when the plant fermented product (2%) was ingested (the overall aging score was significantly low), a tendency that the degree of inflammation is suppressed was observed.

A literature survey was performed on all narrowed down 68 genes. In the case of inflammation, the same gene may work to both increase and decrease, and therefore, it is necessary to confirm the genes individually. A search was performed by inputting "Gene Symbol" and "Inflammation" to Pub-Med and inflammatory symptoms in mammals were surveyed. When the number of hits was extremely large, Google search was also used, and when there were no hits in Pub-Med, "Gene Description" was also used. In addition, citations in Gene Database were also referred to.

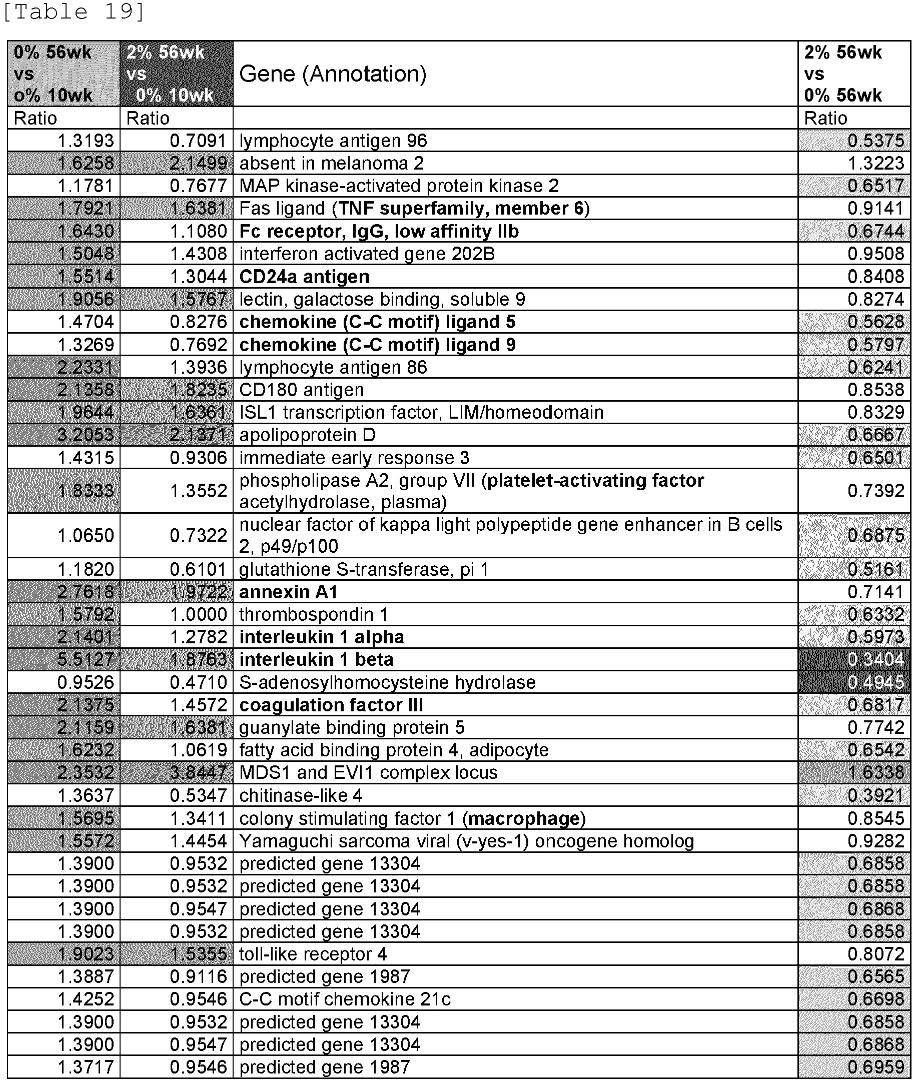

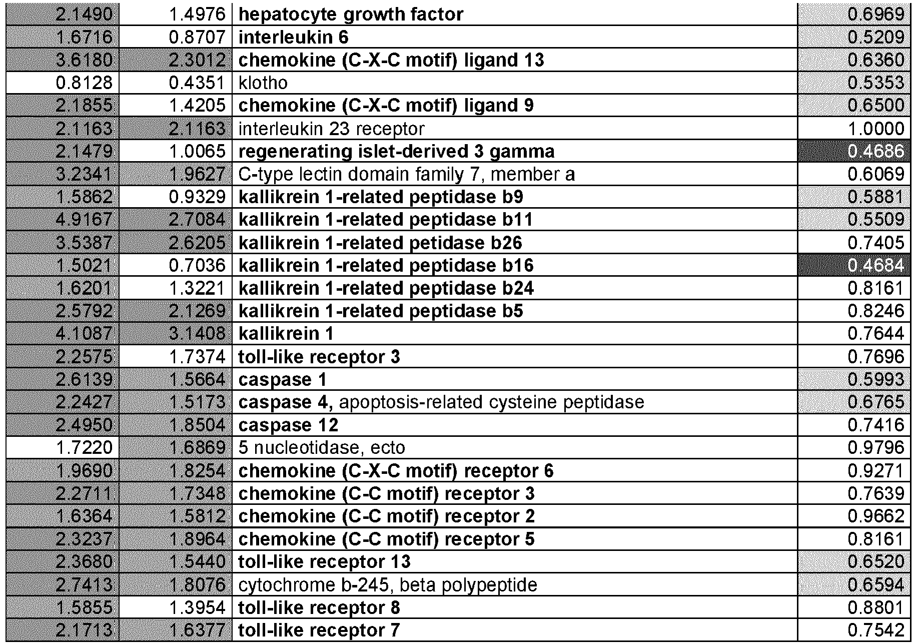

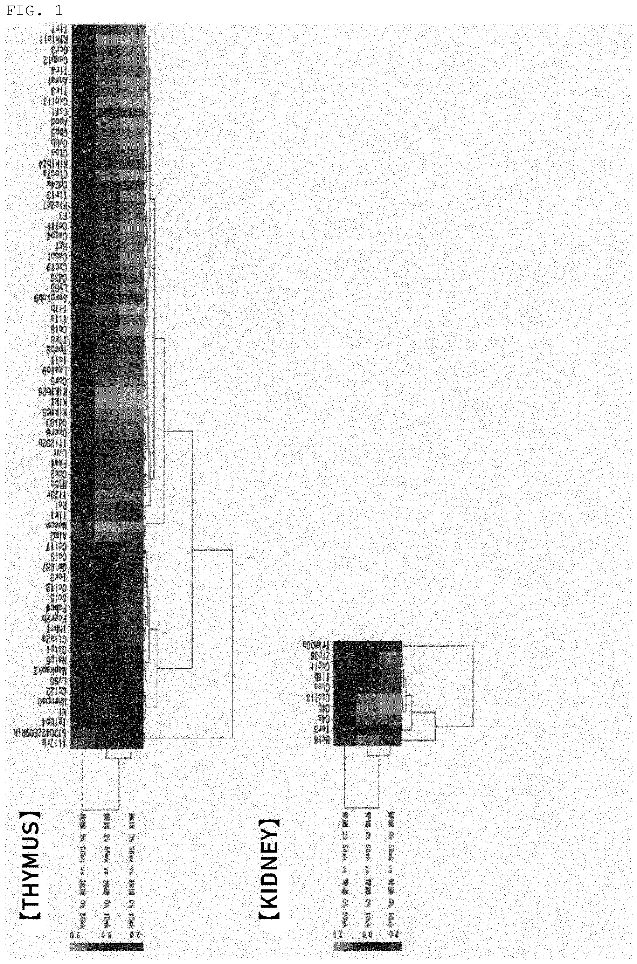

There were 11 out of 68 cases where inflammatory symptoms were suppressed by an increase in gene expression. There were 5 cases where the gene shows both increase and decrease. The comprehensive evaluation of the effect of ingestion of the plant fermented product is shown in the rightmost column. The inflammation suppressing effect was seen in 36 out of 68 cases, and the suppression tendency was seen in 27 cases, and the inflammatory symptoms were enhanced. These included a large number of genes related to chronic inflammation. The heat maps of the DNA microarray analysis of the thymus and the kidney are shown in FIG. 1.

When a case having a clear tendency to suppress inflammation due to ingestion of the plant fermented product was extracted, 15 cases were extracted. As shown in the patent application (published) "PROPHYLACTIC AGENT FOR SPONTANEOUS CANCERS", the incidence ratio of spontaneous cancer was 50% in mice at old age, but there was a significant reduction in spontaneous cancers (17%) in the plant fermented product ingestion group, and it was revealed that the suppression of chronic inflammation is presumed to be involved in the suppression of spontaneous cancers.

Although it leads to exacerbation of inflammatory symptoms by an increase in gene expression, it exhibits an expression suppressing effect in most cases. However, it was also found that the relief of inflammatory symptoms by enhancement of gene expression showed a tissue protecting action, which indicates the versatility of inflammation. For example, the gene Ly86, also known as MD1 (Myeloid differentiation protein 1), has been reported to exacerbate cardiac allergy due to gene deficiency and also to relieve the symptoms of ulcerative colitis, and showed dual properties.

The DNA microarray test was performed also for the kidney. The number of hits in the range of 1.5-fold increase to 0.66-fold decrease (the same conditions as for the thymus) was 10, and the inflammation due to aging was milder as compared to the 68 cases for the thymus. Although it is stated in the material of the SAM study group that SAMP1 (male) develops renal atrophy due to aging, however, the renal weight was also unchanged at the 56 weeks of age, and the glomerular structure was also normal in the histological examination results. Among these 10 cases, the highest value was 1.66 times increase of IL1-β, but it was slight, and it was unchanged in the plant fermented product ingestion group, and a tendency of slight inflammation in the kidney was also suppressed.

### (b) IL1-β-related genes

As shown in the questionnaire results, the plant fermented product exhibits an effect on alleviation of various chronic inflammatory symptoms. The various effects seemed to be exhibited by suppressing inflammatory factors located upstream of the chain response of chronic inflammation. It is apparent from the following paper that IL-1β is an important factor in many inflammations. Therefore, the expression of IL1-β gene was analyzed. The expression increase ratio of IL1-β at 56 weeks of age (sample = 0%) was 5.5 times that of mice at 10 week of age (sample = 0%) which was the largest, but it was 1.9 times and decreased significantly in the plant fermented product ingestion group. The reduction ratio was 0.34 which was the largest when performing comparison between the presence and absence of the plant fermented product in mice at 56 weeks of age.

IL1-β is a central inflammatory mediator and intervenes in many inflammatory responses, and is involved in the expression of inflammatory molecules such as TNF-α and IL-6 via NF-κB, which is an important factor in inflammatory responses, and is located upstream thereof.

The most obvious case of the involvement of IL1-β in inflammation is a specific antibody canakinumab (development number ACZ885), which is an antibody drug developed by Novartis International AG. Efficacy against atherothrombosis, which is the leading cause of acute coronary syndrome and cardiovascular death, has been demonstrated in a large clinical trial enrolled 10,000 or more cases for about 6 years. (CANTOS: Canakinumab Anti-inflammatory Thrombosis Outcomes Study, The Lancet 2017) (https://www.novartis.com). This is an example in which pinpoint suppression of the inflammatory action of IL1-β exhibited effectiveness.

The reports that IL1-β is associated with inflammatory diseases are as follows.
· High expression in epithelial cells in inflammatory chronic obstructive pulmonary disease (Pub-Med)
· Report of proof of pre-inflammatory gene (Pub-Med)
· Report of being an inflammation-related gene for bronchitis

### (Pub-Med)

An IL1-β measurement kit is commercially available as a diagnostic agent. As a disease showing an abnormal value of IL1-β, "chronic fatigue syndrome" is exemplified together with many inflammatory diseases in the measurement in a peripheral monocyte culture supernatant (described in a catalog of Central Laboratory, Falco Biosystems Ltd.).

That is, it is clear that chronic fatigue syndrome and chronic inflammation are overlapping regions.

### (c) Aim2-related gene

The Aim2 gene shows both reduction and decrease in inflammation by the increase in expression.
· Suppression of microbial infection with inflammasomes
Suppression of psoriasis, dermatitis, arthritis, and autoimmunity
· Inflammation due to excess cholesterol is inflammation by an increase in Aim2
• In pancreatitis, inflammation is decreased by a decrease in Aim2

### (d) Pathway analysis

As the genes displayed in Pathway analysis results, genes with 1.5 times or more increase and 0.66 times or less decrease are extracted and displayed. The limit range of this increase or decrease is the same as in the clustering analysis. However, in the clustering analysis, the range is limited by the keyword "inflammation", and therefore, the number of genes in the displayed ranges of both is different, and the number of displayed genes is larger in the Pathway analysis. Therefore, changes in genes which are not displayed in the inflammation clustering analysis can be observed.

Changes in genes in the test groups shown in Table 16 were displayed by the decrease and increase for each pathway. In the case of the sample at 0% and 56 weeks of age, the increase in inflammation-related genes was remarkable, and it was possible to estimate that ingestion of the sample at 2% suppressed inflammation by this comparison of the increase and decrease.

**[Table 20]**

| |
|---|
| Table 16 (1) (decrease) = 32 cases, Table 16 (1) (increase) = 348 cases |
| Table 16 (2) (decrease) = 128 cases, Table 16 (2) (increase) = 145 cases |
| Table 16 (3) (decrease) = 225 cases, Table 16 (3) (increase) = 103 cases |

The phenomenon, which was found in the inflammation clustering analysis, that the inflammation-related gene cluster in the thymus increases by long-term breeding without a sample and the inflammation tends to be suppressed by the ingestion of the plant fermented product was also observed in the Pathway analysis in the same manner. In addition, for IL-6, IL-7, TNF-α, etc., which are important inflammation-related factors that were not extracted by inflammation clustering, an anti-inflammatory effect due to the plant fermented product was observed in the same manner, and further, suppression of lung fibrogenesis that frequently occurs in inflammation was also observed. In addition, Type II interferon, which is an inflammation mediator and showed little change in the inflammation clustering analysis, showed a similar tendency (Table 21).

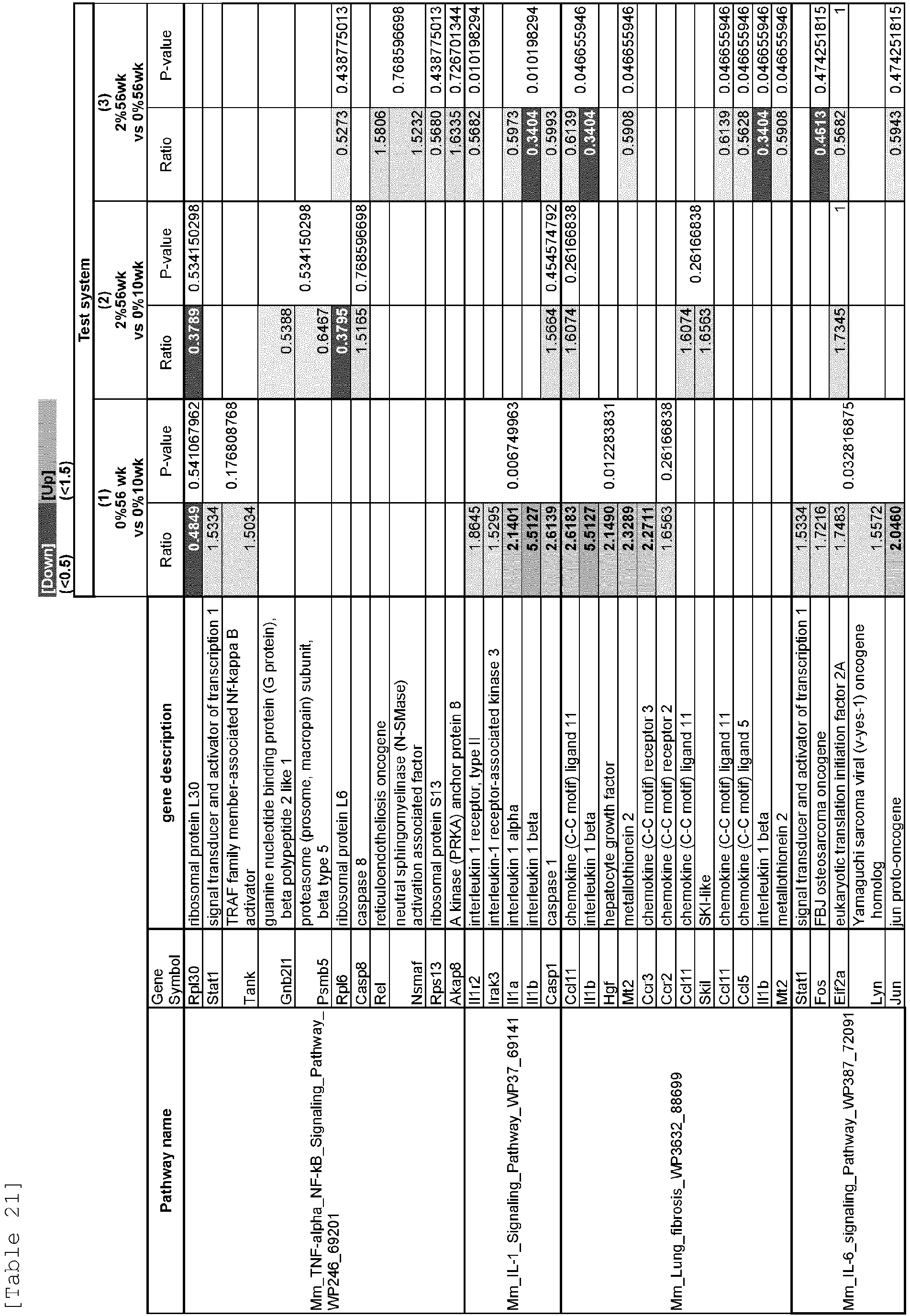

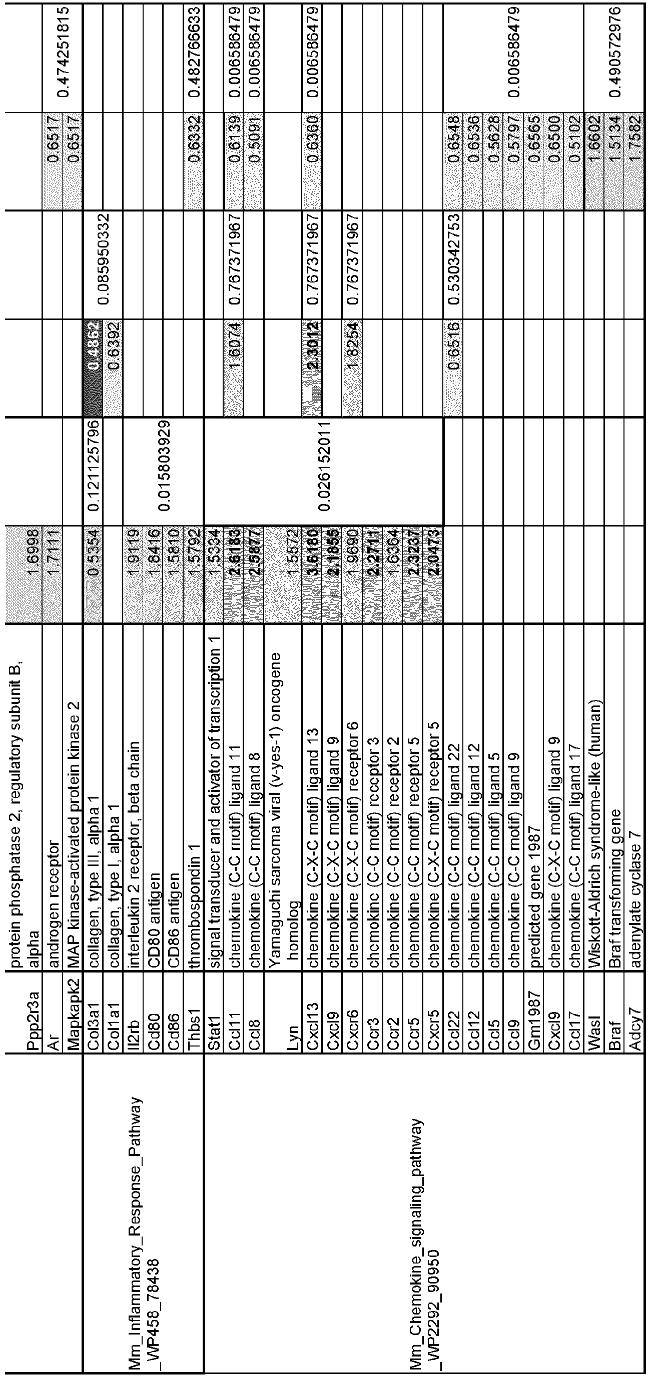

### (1. IL-6)

1) IL-6 Pathway significantly increased (P = 0.033) in the old age group with accelerated senescence at 56 weeks of age as compared to the young mice at 10 weeks of age. Seven genes involved were found. Most are found in Pathway Map.
2) In the 2% plant fermented product ingestion group, the increase in 6 out of 7 types disappeared. In Gene Symbol, Elf2a did not decrease.
3) The plant fermented product suppressed Pathway of IL-6, which is an inflammatory cytokine.
4) In the inflammation-related review, IL-6-related genes Jak1, Jak2, Stat1, and Stat3 have been attracting attention as targets for drug development, but they were not found in this table.

However, there is also a possibility that another Pathway may exist because the plant fermented product is highly effective in patients with rheumatoid arthritis in which IL-6 is strongly involved.
5) There is no approved drug for severe cases due to cytokine storm in acute respiratory distress syndrome (ADRS) in severe cases of COVID-19.
6) A case where "tocilizumab", which is an IL-6 inhibitor, is effective was observed, and a clinical trial is still continued.

### (2. Lung fibrosis)

1) Pathway analysis was performed for lung fibrogenesis derived from lung inflammation.
2) Fibrosis is derived from the fibrogenesis of a tissue after inflammation (in order to repair the tissue damaged by the inflammation, fibers proliferate cells to achieve the repair).
3) Significantly strong gene expression occurs at P = 0.01, which is suppressed by the plant fermented product at 2%. IL-1β exhibits the most significant increase and significant suppression.

### (3. TNF-α)

1) TNF-α is a representative of inflammatory substances and is directly involved in many inflammations. However, it was presumed that it is not deeply involved in the aging of SAM mice because the P value of this Pathway is not significant. It is known that TNF-α is less involved in rheumatoid arthritis against which the plant fermented product exhibited high efficacy. IL-6 is located more upstream in the inflammatory response than TNF-α and has more diverse response regulatory actions.

### (4. IL-1)

1) IL-1 is also an important cytokine that causes inflammation. In the Pathway analysis, the P value showed P = 0.007 and P = 0.01 in any test system, and IL-1 is one of the main protagonists of the inflammatory response in this test. The plant fermented product has an action of suppressing the expression of most of the genes involved in this Pathway. It matched well with the keyword search data. However, it often works downstream of IL-6 in the inflammatory response.

### (5. Chemokaine)

1) A chemokine is a basic protein that expresses its action via a G protein-coupled receptor and is a group of cytokines. It induces migration of leukocytes or the like and is involved in the formation of inflammation. It means a chemotactic cytokine. It causes various inflammatory responses at the site of inflammation and is located downstream of IL-6.
2) A large number of chemokines were detected also in this Pathway, and a P value is 0.02 and is significant. They increased in Test System 1 (aging) and were all decreased by the ingestion of the plant fermented product.

### (6. Comprehensive findings)

1) The most notable was IL-6.
2) The P value was 0.03, and it increased in Test System 1 (aging) and suppressed by the ingestion of the plant fermented product as shown in Test Systems 2 and 3.
3) A Janus kinase (JAK) inhibitor is attracting attention as the target of an anti-rheumatic agent. It has been shown in the course of treatment trials that the suppression of IL-6, which is one of the inflammatory factors by this inhibitor, suppresses severe viral pneumonia in COVID-19 (new coronavirus infection), which is expected to lead to suppression of exacerbation, and clinical trials are currently underway. This plant fermented product also exhibited high efficacy in patients with rheumatoid arthritis in questionnaire, and also, it was shown by the doctor's diagnosis that the plant fermented product is effective also in patients with Behcet's disease, which is a similar disease. It is known that in these two diseases, the blood concentration of IL-6 increases with the exacerbation of the disease state, and decreases with sedation.
4) Two types of medicinal products by gene recombination for the IL-6 receptor have currently been approved. However, both have high specificity and do not target diverse inflammatory responses. Moreover, these are an injection (intravenous infusion), and are instructed to use only for serious patients because the drug price is very high.
5) On the other hand, this plant fermented product is a food product and is widely sold in a commodity market, and very few side effects or discontinuation cases have been reported. Moreover, it is less expensive than the medicinal product. As also shown in the list of inflammation-related Pathways, not only the suppression of IL-6, which is the main protagonist of chronic inflammation, but also the suppression of IL-1, a chemokine, and lung fibrogenesis resulting from inflammation occurs, and is diverse. Inflammatory responses, especially chronic inflammatory responses are diverse, and it can suppress many inflammatory factors that also take charge of controlling normal biological defense responses. Conventional medicinal products that suppress inflammation with a 1:1 action may also suppress normal biological defense responses in some cases. The plant fermented product has a normalization function with few side effects while keeping the overall balance like a Kampo medicine. Moreover, it is an oral preparation and is inexpensive.

It is apparent that a wide range of inflammatory symptoms are exhibited in parallel with various aging symptoms due to getting old, and that the ingestion of the plant fermented product achieves the suppression of various inflammatory symptoms in parallel with the suppression of aging symptoms. Carcinogenesis and metastasis are scary to many people and affect mental QOL. Chronic inflammation is the basis of all so-called lifestyle-related diseases such as diabetes mellitus, obesity, and hyperlipidemia, aging, etc. and the suppression of chronic inflammation is effective in extending healthy life expectancy.

From the above results, it was proved that the plant fermented product improves QOL related to inflammation.

### Example 3

Support for QOL improvement based on chronic fatigue:

### (1) Mice

The same mice as in Example 2 were used.

### (2) Sample

The same samples as in Example 2 were used.

### (3) Collection of specimens

It was performed in the same manner as in Example 2.

### (4) DNA microarray

The following gene-related expressions were analyzed based on the analytical data (the number of genes: 566) obtained from the above institution.

### (a) Interferon-related genes

As side effects of interferon on humans, malaise, weakness, and fever frequently occur. Also in the inflammation clustering analysis data, the Type II interferon gene was increased with aging, and suppression of the expression was observed by ingestion of the plant fermented product. The inflammation clustering analysis also shows similar results, although the number of target genes is small. This suggests a link between inflammation and chronic fatigue.

### (b) Pathway analysis

With respect to chronic fatigue, even when a keyword (fatigue, tired, exhaust, anxiety) search was performed in the clustering analysis, the number of hits was 0. Therefore, WIKI-Pathway analysis was used. Since a database different from that of the clustering analysis was used, there may be differences in the degree of increase or decrease and the genes to be hit.

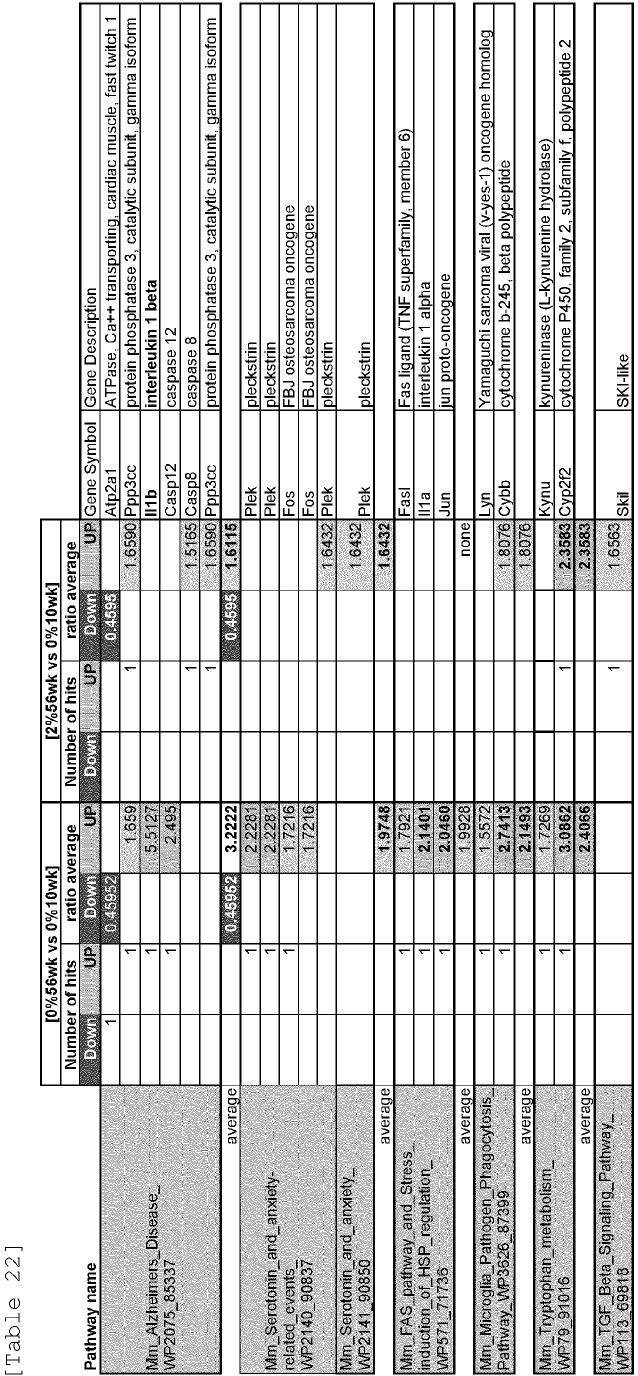

In the Pathway analysis, the involvement of genes related to Alzheimer's disease was observed, and the involvement of the gene decrease "Atp2a1 = ATPase, Ca membrane permeation" caused apoptosis due to the decrease in Ca²⁺ in nerve cells, and recently, the involvement in Alzheimer's disease is suspected. The suppressing effect of the plant fermented product is not remarkable, and it may be a phenomenon associated with aging. As for the increase in the expression of the caspase 12 gene, which is one of the markers associated with inflammation, the change disappears by the ingestion of the plant fermented product. What is noteworthy is interleukin 1-β. In the case of 0% and 56 weeks of age, the expression level reached 5.5 times that in the mice at young age, but in the plant fermented product ingestion group, no increase or decrease was observed. In the inflammation clustering, the expression level decreases to about twice by the ingestion of the plant fermented product. It is known that IL1-β is secreted when microglia in the brain are activated by infection or stress, moves to the whole body and causes inflammation, and also develops symptoms of chronic fatigue syndrome.

It is known that serotonin and its precursor, tryptophan are associated with chronic fatigue, which has recently changed a bit. In the Pathway analysis, both genes increased 2 to 3 times in the old age group, but decreased in the plant fermented product ingestion group. Association with the alleviation of chronic fatigue symptoms is suggested. Two types of microglial genes also showed a similar tendency.

Similar to inflammation, also genes involved in chronic fatigue symptoms decreased their gene expression in the plant fermented product ingestion group.

From the above results, it was proved that the plant fermented product improves QOL related to dementia such as Alzheimer's disease and chronic fatigue.

### Example 4

Support for regulation of enteric bacteria:

### (1) Analysis of enteric bacteria in senescence-accelerated mice

The test groups of SAM mice used are shown in Table 17. Therefore, the results of DNA microarrays can be compared with each other at the individual level. Note that in the microarray analysis, stool samples stored in the test of the thymus of 6 mice in each group were used. The analysis of enteric bacterial flora was outsourced to TechnoSuruga Laboratory Co., Ltd. (Shimizu City) based on the collected individual stools, and analysis was performed up to the strain the TFLP method (human type and mouse type) and a next-generation sequencer (human type) using 16sRNA.

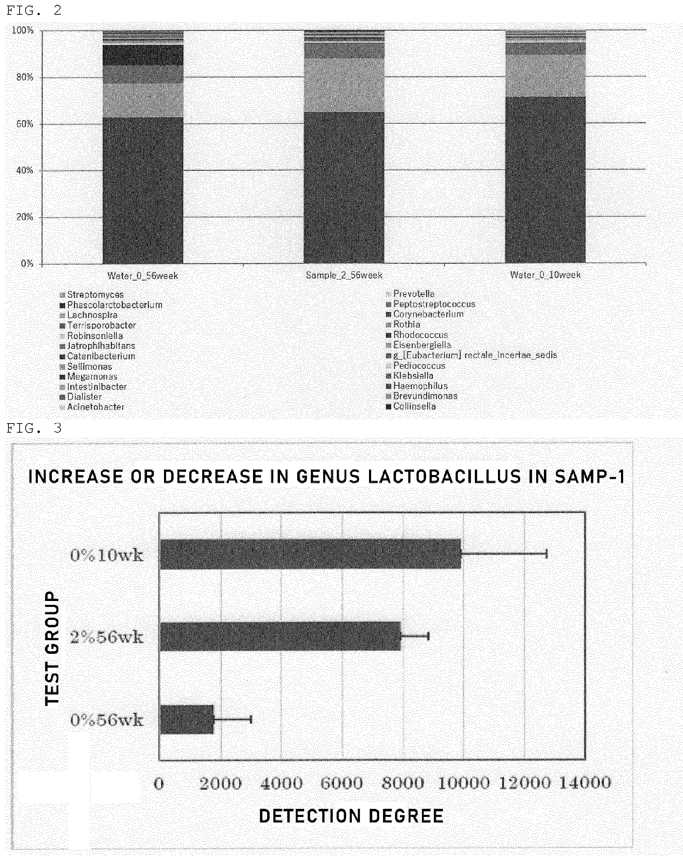

The abundance ratio at the genus level is shown in FIG. 2. Note that since a human type gene was used as a template, some were determined as "rejected". An increase or decrease in each test group for the top three microorganisms, Lactobacillus, Bacteroides, and Clostridium is shown in FIGS. 3 to 6. The maximum value of each bar graph shows one classified as "rejected", but among the enteric bacteria excluding this, in the case of ingestion of a water sample, the genus Lactobacilli showed a lower value at 56 weeks of age than at 10 weeks of age, however, in the 2% plant fermented product ingestion group, even at 56 weeks of age, the genus Lactobacilli did not decrease and showed a value close to the value at 10 weeks of age. This matches with the results in the literature ("Effects of Enteric Bacterial Flora and Immune System on Emotions", Tomoo Miyajima, "Experimental Medicine Vol. 37, (2) 140-147 (2019)"). The ingestion of the plant fermented product suppresses the intestinal aging. Although the stool was collected after dissection, the elasticity of the intestinal tract itself, peritoneum, arteries/veins, etc. was higher in the sample ingestion group, and was close to the elasticity of young mice at 10 weeks of age. Although there is no Bifidobacterium in mice, the genus Lactobacillus is a major enteric bacterium, and the results that it decreased in the mice at old age as compared to the mice at 10 weeks of age, and did not decrease by the ingestion of the plant fermented food suggests that it also works to suppress the intestinal aging.

Although there are many bacteria of the genus Lactobacillus in mice at young age, the genus Lactobacillus decreased sharply in the case of the sample at 0% and 56 weeks of age, but the decrease was suppressed in the 2% administration group. From FIG. 3, the genus Lactobacillus with the highest detection frequency was analyzed down to the strain level. In the analysis of Lactobacillus at the strain level, there are mostly 3 strains, and L. reuteri and L. intestinalis account for most, and in the case of 0% and 56 weeks of age, their detection frequency was lower than in the case of 10 weeks of age, and decreases with aging, and in the plant fermented product ingestion group, the decrease is suppressed even at the old age. This matches with the results of the questionnaire for humans that the improvement of bowel movement was observed at a high rate in those who ingested the plant fermented product. L. reuteri is widely commercially available as a useful lactic acid bacterium, and its usefulness is detailed in Wikipedia.

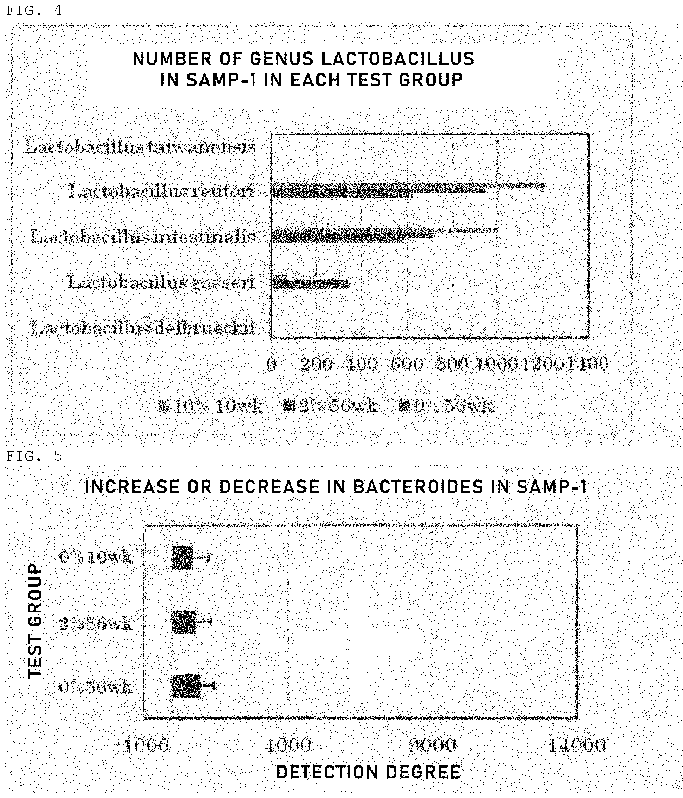

In FIG. 5, almost no increase or decrease in Bacteroides was observed.

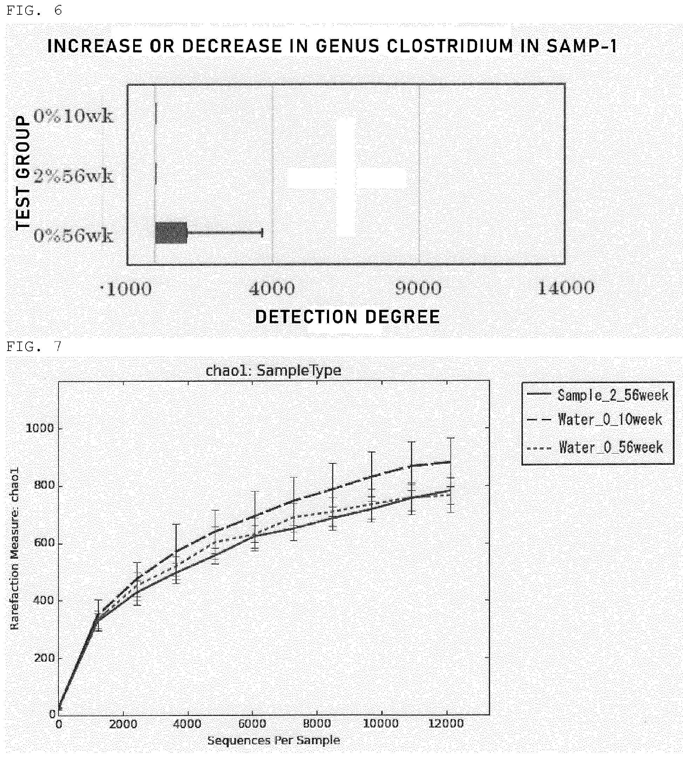

In FIG. 6, Clostridium subclass changed less than the genus Lactobacillus.

### (2) Analysis of diversity of aging of SAM mice and changes in enteric bacterial flora

Arai et al. explain association of aging and enteric bacterial flora ("Enteric Flora and Aging", Japanese Journal of Geriatrics, Vol. 53, 318-325, 2016). According to this literature, it is known that the immune function, particularly the response of acquired immunity decreases with aging, and it is called immunosenescence. The immunosenescence induces a mild chronic immune response, "inflamaging". The enteric bacterial flora of the elderly has a larger individual difference, and also is less diverse and stable as compared to that of an adult human. In the long-term breeding test of SAM mice, although Lactobacillus was detected more frequently in the young mice, the detection frequency decreased to about 50% in the mice at old age, but was maintained at about 82% in the 2% plant fermented product ingestion group. This was remarkable in L. reuteri, and this bacterium is known to have an immunopotentiating action. Here, analysis was performed by focusing on the changes in diversity of bacterial flora due to aging.

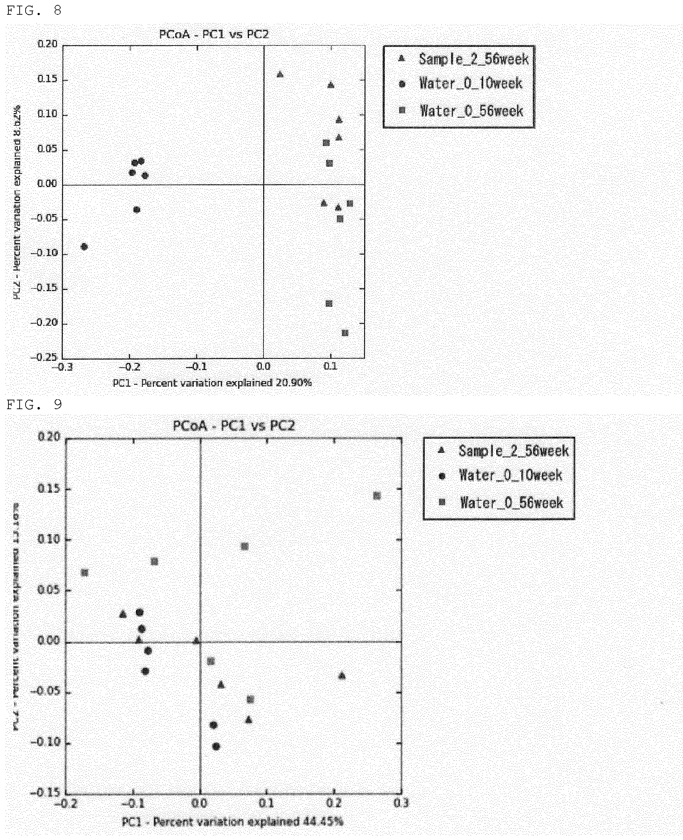

With respect to diversity, both α-diversity and β-diversity were examined using Unifrac analysis. The results are shown in FIGS. 7 and 8.

In the α-diversity analysis, mice at 10 weeks of age (0%) were superior to mice at 56 weeks of age (0%/2%) in diversity, which matched the results in the above literature, but did not reach p = 0.05 and there was no significant difference. In other words, the three groups used in the α-diversity analysis showed similar diversity.

In the β-diversity analysis, there are an Unweighted method that does not consider the number of reads (composition ratio) contained in OUT and a Weighted method that considers it. The former reflects only the presence or absence of bacterial strain, and the latter reflects the difference in composition ratio of the same bacterial strain in the distance of similarity between two bacterial floras ("Latest technology for human intestinal microbiome analysis", Masahira Hattori, Jpn. J. Clin. Immunol., Vol. 37, (5) 412-422 (2014)).

In the Unweighted-unifrac analysis, as shown in FIG. 8, in the test group of 10 wk 0% (●), 6 points of 6 mice were compactly distributed at the left edge, but the points of all mice at 56 weeks of age gathered vertically at the right edge, which was apparently different from those of the mice at 10 weeks of age. Further, at 56 weeks of age, in the 2% group, the points were compactly distributed in the first quadrant, and in the 0% group, the points were slightly dispersed and distributed in the second quadrant. It shows that there is a difference in diversity of the two, but the composition ratio of OUT is not taken into consideration. FIG. 9 which shows the Weighted Unifrac analysis in which the number of reads (composition ratio) between bacterial floras is taken into consideration is shown.

The points of the young mice fed with 0% at 10 weeks of age (●) were distributed in a straight line with a downward slope, but the points of the mice fed with 0% at 56 weeks of age were widely dispersed, which indicates that the difference in bacterial flora among mice was large. On the other hand, even at 56 weeks of age, the distribution of the bacterial flora of the mice fed with the sample at 2% (1) was gathered, and similarity to the slope of the distribution of that of the mice at 10 weeks of age was observed. The SAM mice at old age fed with the plant fermented product at 2% show a tendency similar to the young mice in terms of β-diversity of enteric bacteria. The relationship between these three had a similar tendency to other chronic inflammation indices and indices of chronic fatigue.

Although the tendency is similar to the dispersion chart at the genus level, the points of the mice at 56 weeks of age are distributed on the right side when the sample concentration was 0% and 2%, and the dispersion tendency is different from that of the young mice (at 10 weeks of age). In the 2% group, the variation among mice is small.

In this manner, in the in vivo effect of the plant fermented product, aging of SAM mice was suppressed, the lifespan was extended, further, spontaneous cancer was suppressed, and an immune checkpoint suppressing effect was exhibited. It is considered to be a rare fermented product that comprehensively leads to the extension of healthy life expectancy and moreover has an effect of comprehensively improving the quality of life. As for changes in enteric bacteria in aged mice, a decrease in the genus Lactobacillus occurred as compared to mice at young age, and it was also supported by the fact that the decrease in Lactobacillus is prevented by the ingestion of the plant fermented product. It has become clear that it also has a so-called prebiotics effect.

From the above results, since the plant fermented product significantly increases the genus Lactobacillus, especially Lactobacillus reuteri, it was proved that the plant fermented product can be widely used for the purpose of adjusting the intestinal environment, and suppressing functional gastrointestinal disorders including diarrhea and constipation, and the growth of Helicobacter pylori, dental caries bacteria, or periodontal disease bacteria, etc.

### Example 5

Human administration test:

### (1) Method

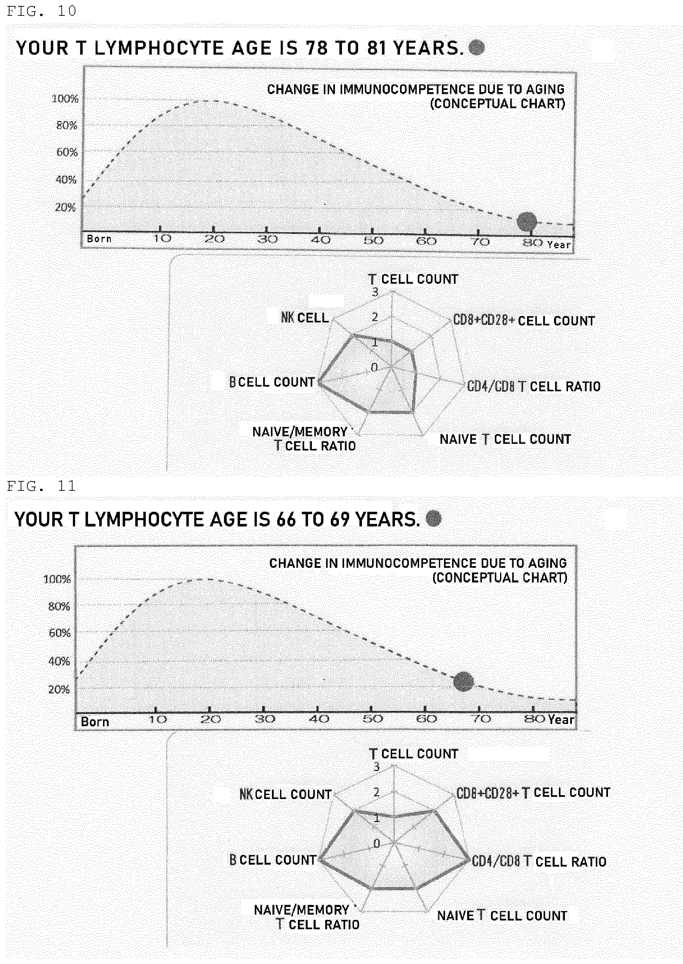

A plant fermented product (Amou Koso Kinjirushi) was administered to a 69-year-old man (n = 1) at a dose of 5 g/day. The ingestion of immune-related foods such as yogurt and banana was stopped for 2 weeks before administration, and the administration was started. The administration was performed daily for 4 weeks, and the peripheral blood was collected at a total of 3 time points: the completion of administration and 2 weeks after the completion, and used for a lymphocyte test. The analysis was outsourced to Orthomedico, Inc. The outline of the method is described on the homepage of Orthomedico, Inc. (https://www.orthomedico.jp/clinical-trials/case/immunity.html). At the same time, stool was collected and used for analysis of enteric bacteria, and the analysis was outsourced to Orthomedico, Inc. The TFLP method was used as the analysis method. The results are shown in FIGS. 10 to 12.

Immediately before ingestion, T cells were present sufficiently, but the CD4+/CD8+ cell ratio and the CD8CD28+ T cell count were low. The T lymphocyte age was evaluated to be 78 to 81 years (Fig. 10).

After the ingestion for 4 weeks, the CD4+/CD8+ cell ratio and the CD8CD28+ T cell count increased. The T lymphocyte age was evaluated to be 66 to 69 years (Fig. 11).

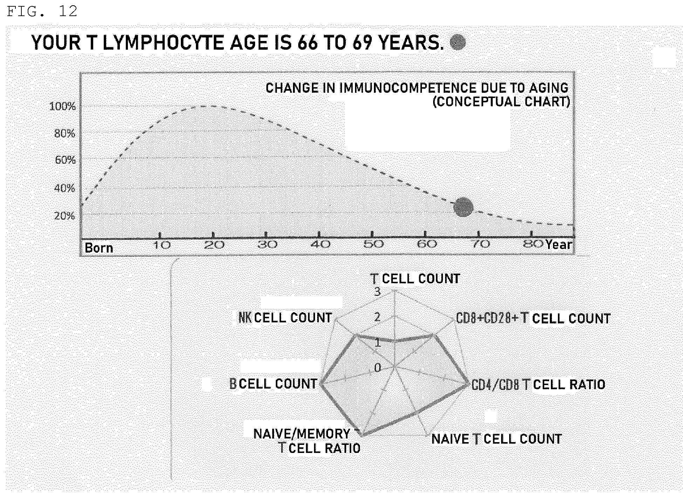

Two weeks after the completion of ingestion, the lymphocyte pattern was maintained. The T lymphocyte age was evaluated to be 66 to 69 years (Fig. 12).

Based on the above results, the ingestion of the plant fermented product by a human (69-year-old man) was attempted. According to the method of estimating the immune age from the trend of peripheral blood lymphocytes before, immediately after, and 4 weeks after the daily ingestion for 4 weeks, the value corresponding to 71 to 81 years old and 80 years old before the ingestion decreased to a value corresponding to 66 to 69 years old by the ingestion, and the value was maintained even 2 weeks after the discontinuation of the ingestion. According to Yamakoshi, the relationship among SASP caused by cellular senescence, chronic inflammation, and carcinogenesis is explained ("Cellular Senescence and Chronic Inflammation" Japanese Journal of Geriatrics, Vol. 53, pp. 88-94 (2016)). At this time, when the analysis of enteric bacteria was performed, an increase in Clostridium subcluster IXa suggesting immunity enhancement was observed.

From the above results, the plant fermented product recovered the aging of lymphocytes in peripheral blood and showed suppression of immunosenescence. The enteric bacteria and the increase in Clostridium cluster Xa, which is involved in immunity enhancement, also support it. Immunosenescence is deeply involved in antigen-antibody reaction ability such as an infection defense ability, and it is one of the indices of immunosenescence taken up in the QOL evaluation axes.

### Example 6

Improvement of QOL based on dementia:
A patient who received a definitive diagnosis of Alzheimer's disease from MRI imaging diagnosis or the like daily ingested a plant fermented product (Amou Koso Kinjirushi) at a dose of 5 g/day. One month later, family and friends noticed a change in the patient's facial expression. The eyes became more focused, the facial expressions brightened, and the patient was able to actively participate in conversation. After that, the improved state continued for another month.

When diagnosis was made again a little over two months after ingestion, from the MRI image, the patient was diagnosed with right cerebral atrophy but no Alzheimer's disease or hydrocephalus (there was no mention of hippocampus).

Further, the improved state continued even after several months had passed since ingestion.

### Example 7

Miso-like food for improving QOL:
The paste-like plant fermented product produced in Production Example 1 and miso were mixed, and the taste was adjusted with a seasoning, whereby a miso-like food for improving QOL was produced.

### Example 8

Drink for improving QOL:
The paste-like plant fermented product produced in Production Example 1 was dissolved in water, and then, mixed with a fructose-glucose syrup and a fruit juice to adjust the taste, whereby a drink for improving QOL was produced.

### Example 9

Soft capsule:
A mixture obtained by mixing the paste-like plant fermented product produced in Production Example 1 with a vegetable oil and lecithin was filled in a soft capsule by a conventional method, whereby a soft capsule used for improving QOL was produced.

### Example 10

Chewable tablet:
A mixture obtained by mixing the paste-like plant fermented product produced in Production Example 1 with crystalline cellulose and white soft sugar was tableted by a conventional method, whereby a chewable tablet used for improving QOL was produced.

### Example 11

Soft capsule:
A mixture obtained by mixing the paste-like plant fermented product (daily dose) produced in Production Example 1 with a vegetable oil, lecithin, and 900 mg of anserine was filled in a soft capsule by a conventional method, whereby a soft capsule used for improving QOL was produced.

### Example 12

Prevention and treatment of IL-6 related diseases:
In the administration test and collection of questionnaire in Example 1, there were cases of improvement of the following diseases.

**[Table 23]**

| Classification of diseases | Gender/age | Conditions | Ingestion amount | Effect |
|---|---|---|---|---|
| Rheumatoid arthritis | female/70 | At the age of 24, rheumatoid arthritis was developed in right leg, the lesion was enlarged one after another and pelvic bone grafting was performed three times. The pain disappeared after starting eating the plant fermented product. Even the attending physician was surprised. | Doubled before 3rd surgery Estimate: 10.2 g/day | highly effective |
| | female/61 | had hard time with rheumatism for many years, but stiffness in hands disappeared, coldness of hands also disappeared, and now it became possible to write with a ballpoint pen. Life got brighter. | unknown | effective |
| | female/64 | Rheumatoid pain was relieved. | unknown | effective |
| | female/70 | Due to chronic rheumatoid arthritis, inflammation of fingers and wrist joints was often caused. After eating enzyme, it became possible to squeeze dustcloth, however lightly. | unknown | effective |
| | male/75 | Started eating due to rheumatism, but still no change. (5 months) | unknown | undeterminable |
| Behcet's disease | female/71 | Diagnosed with Behcet's disease 32 years ago, and later also identified as idiopathic thrombocytopenic purpura. (both are specific diseases). By drinking enzyme, recovery was achieved and the test data decreased, and the specific diseases were dismissed. | 6.9 g/day | determined as effective by doctor |

**[Table 24]**

| Classification of diseases | Gender/age | Conditions | Ingestion amount | Effect |
|---|---|---|---|---|
| Lung inflammation | female/56 | When a patient with bleeding due to suction of sputum was asked to use it, the affected area was cleaned and the patient was grateful. → chronic inflammation | unknown | Relief after bleeding |
| Bronchitis | female/61 | bad bronchi, wheezing, chest pain, and heavy cough. Three months after starting to drink the enzyme, wheezing stopped and cough subsided in three months. | unknown | Cough caused by bronchitis subsided |
| Pneumonia | male/76 | Shadow in chest x-ray disappeared | unknown | Shadow in lung disappeared |
| Pneumonia | male/69 | It was said that there was shadow in X-ray examination, but it disappeared after 10 days. pneumonia? (total?) was confirmed. | 580.3 g/day | Shadow in lung disappeared |
| Bronchitis | male/67 | Chronic bronchitis attacks occurred less. | unknown | |
| Lung inflammation | male/67 | Sputum comes out 5 to 6 times at night and in the morning. (The sputum was like a black candy, but it became white and smooth.) | unknown | Bleeding disappeared |
| Pneumonia | female/58 | lungs were clean in examination at hospital (ingestion amount is large → need checking) 2 bottles/day? | 145.8 g/day | Shadow in lung disappeared |
| Pulmonary emphysema | male/76 | having emphysema, but getting better. could breathe easier | unknown | Breathing was improved |

As described above, the plant fermented product, which is the active ingredient of the quality-of-life improving agent of the present invention, is expected to have effectiveness against rheumatoid arthritis and Behcet's disease, which are autoimmune diseases, and in which the IL-6 concentration in the blood increases in parallel with the exacerbation of the disease state. These are both intractable diseases, and the QOL is significantly reduced due to pain and disease state, and the psychological damage is also great, and the QOL improving effect is high. Further, it has been shown in Example 2 that the plant fermented product, which is the active ingredient of the quality-of-life improving agent of the present invention, suppresses the expression of IL-6, and such a substance that suppresses the expression of IL-6 is unprecedented and is a novel invention. The mechanism is different from that of existing antibody drugs, and therefore, it can also be used in combination with antibody drugs.

Further, as described above, the plant fermented product, which is the active ingredient of the quality-of-life improving agent of the present invention, is expected to have effectiveness also against lung inflammation-related diseases. Such lung inflammation-related diseases are diseases also caused by acute exacerbation due to new coronavirus (COVID-19) infection. Therefore, the plant fermented product, which is the active ingredient of the quality-of-life improving agent of the present invention, is highly likely to suppress the acute exacerbation due to new coronavirus infection, and it is considered that the ingestion from the early stage of infection is effective.

### Industrial Applicability

The quality-of-life improving agent of the present invention can be used to improve a wide range of QOL, for example, QOL based on emotions/behaviors, geriatric symptoms, digestive symptoms, chronic fatigue, chronic inflammation, metabolic diseases, dementia, or the like.

## Claims

1. A quality-of-life improving agent, **characterized by** comprising, as an active ingredient, a plant fermented product that is a mixture of the following substances (a) to (g):
(a) a koji mold fermented product of one type or two or more types of beans/grains selected from the group consisting of barley, black soybean, red rice, black rice, red bean, adlay, Japanese barnyard millet, foxtail millet, and proso millet;
(b) a yeast and/or lactic acid bacterium fermented product of one type or two or more types of fruits selected from the group consisting of mandarin orange, grape, apple, crimson glory vine, peach, persimmon, papaya, pear, watermelon, Prunus mume, fig, Chinese quince, pumpkin, kumquat, yuzu, loquat, apricot, jujube, chestnut, silver vine, and Prunus salicina;
(c) a yeast and/or lactic acid bacterium fermented product of one type or two or more types of root vegetables/tubers selected from the group consisting of purple sweet potato, Jerusalem artichoke, carrot, onion, sweet potato, taro, wild yam, daikon, red turnip, burdock, lotus root, yacon, lily bulb, threeleaf arrowhead, ginger, garlic, and turmeric;
(d) a yeast and/or lactic acid bacterium fermented product of one type or two or more types of flowers/leafy vegetables selected from the group consisting of cabbage, shiso, mulberry leaf, fish mint, mugwort, Sasa veitchii, and dandelion;
(e) a yeast and/or lactic acid bacterium fermented product of one type or two or more types of seaweeds selected from the group consisting of kombu, wakame, and mozuku;
(f) a yeast and/or lactic acid bacterium fermented product of one type or two or more types of seeds selected from the group consisting of black sesame, walnut, and ginkgo nut; and
(g) a yeast and/or lactic acid bacterium fermented product of one type or two types of mushrooms selected from the group consisting of hen-of-the-woods and shiitake.

2. The quality-of-life improving agent according to claim 1, wherein the plant fermented product contains amino acids having the following composition per 100 g of the plant fermented product:
| | |
|---|---|
| arginine | 0.2 to 0.6 g, |
| lysine | 0.1 to 0.7 g, |
| histidine | 0.1 to 0.4 g, |
| phenylalanine | 0.2 to 0.8 g, |
| tyrosine | 0.1 to 0.6 g, |
| leucine | 0.3 to 1.2 g, |
| isoleucine | 0.2 to 0.8 g, |
| methionine | 0.05 to 0.30 g, |
| valine | 0.2 to 0.9 g, |
| alanine | 0.2 to 0.9 g, |
| glycine | 0.2 to 0.7 g, |
| proline | 0.4 to 1.2 g, |
| glutamic acid | 1.2 to 3.0 g, |
| serin | 0.2 to 0.8 g, |
| threonine | 0.2 to 0.7 g, |
| aspartic acid | 0.4 to 1.5 g, |
| tryptophan | 0.03 to 0.15 g, and |
| cystine | 0.05 to 0.40 g |

3. The quality-of-life improving agent according to claim 1 or 2, wherein the plant fermented product contains organic acids having the following composition per 100 g of the plant fermented product:
| | |
|---|---|
| citric acid | 0.5 to 1.2 g |
| malic acid | 0.05 to 0.5 g |
| succinic acid | 0.04 to 0.3 g |
| lactic acid | 0.5 to 6.0 g |
| formic acid | 0.01 to 0.1 g |
| pyruvic acid | 0.005 to 0.05 g, and |
| free γ-aminobutyric acid | 0.01 to 0.05 g |

4. The quality-of-life improving agent according to any one of claims 1 to 3, wherein the lactic acid bacterium is one strain or two or more strains selected from the group consisting of Pediococcus acidilacti (P. acidilacti), Lactobacillus brevis (L. brevis), Leuconostoc mesenteroides (L. mesenteroides), Lactobacillus plantarum (L. plantarum), Lactococcus lactis (L. lactis), Lactobacillus sake! (L. sakei), Pediococcus pentosaceus (P. pentosaceus), Lactobacillus casei (L. casei), and Lactobacillus curvatus (L. curvatus).

5. The quality-of-life improving agent according to any one of claims 1 to 4, wherein the yeast is Saccharomyces cerevisiae (S. cervisiae) and/or Zygosaccharomyces rouxii (Z. rouxii).

6. The quality-of-life improving agent according to any one of claims 1 to 5, which improves the quality of life based on emotions/behaviors.

7. The quality-of-life improving agent according to any one of claims 1 to 5, which improves the quality of life based on chronic fatigue.

8. The quality-of-life improving agent according to any one of claims 1 to 5, which improves the quality of life based on geriatric symptoms.

9. The quality-of-life improving agent according to any one of claims 1 to 5, which improves the quality of life based on digestive symptoms.

10. The quality-of-life improving agent according to any one of claims 1 to 5, which improves the quality of life based on metabolic diseases.

11. The quality-of-life improving agent according to any one of claims 1 to 5, which improves the quality of life based on dementia.

12. The quality-of-life improving agent according to any one of claims 1 to 5, which improves the quality of life based on chronic inflammation.

13. The quality-of-life improving agent according to claim 12, wherein the improvement of the quality of life based on chronic inflammation is based on suppression of IL-6 expression.

14. The quality-of-life improving agent according to claim 12, wherein the chronic inflammation is rheumatoid arthritis, juvenile idiopathic arthritis, Castleman's disease, adult-onset Still's disease, or cytokine storm.

15. A food or drink for improving the quality of life, comprising the quality-of-life improving agent according to any one of claims 1 to 14.
